(19) Europäisches Patentamt
European Patent Office
Office européen des brevets

(11) EP 2 941 445 B1

# (12) EUROPEAN PATENT SPECIFICATION

(45) Date of publication and mention of the grant of the patent:
**22.03.2017 Bulletin 2017/12**

(21) Application number: **14706130.3**

(22) Date of filing: **07.01.2014**

(51) Int Cl.:
*C08F 220/18* $^{(2006.01)}$
*C08F 220/30* $^{(2006.01)}$
*C08F 220/38* $^{(2006.01)}$
*C08L 33/08* $^{(2006.01)}$
*C08L 33/10* $^{(2006.01)}$
*G02B 1/00* $^{(2006.01)}$
*C08F 220/10* $^{(2006.01)}$

(86) International application number:
**PCT/IN2014/000015**

(87) International publication number:
**WO 2014/106866 (10.07.2014 Gazette 2014/28)**

## (54) FLEXIBLE, HIGH REFRACTIVE INDEX HYDROPHOBIC COPOLYMER

FLEXIBLES HYDROPHOBES COPOLYMER MIT HOHEM BRECHUNGSINDEX

COPOLYMÈRE SOUPLE HYDROPHOBE À INDICE DE RÉFRACTION ÉLEVÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **07.01.2013 IN DE37652012**

(43) Date of publication of application:
**11.11.2015 Bulletin 2015/46**

(73) Proprietor: **Council of Scientific & Industrial Research**
**New Delhi 110 001 (IN)**

(72) Inventors:
- **PONRATHNAM, Surendra**
  **Pune-411 008**
  **Maharashtra (IN)**
- **GHORPADE, Ravindra, Vasant**
  **Pune-411 008**
  **Maharashtra (IN)**
- **CHAVAN, Nayaku, Nivrati**
  **Pune-411 008**
  **Maharashtra (IN)**
- **RAJDEO, Kishor, Sudam**
  **Pune-411 008**
  **Maharashtra (IN)**
- **BHONGALE, Sunil, Sitaram**
  **Pune-411 008**
  **Maharashtra (IN)**

(74) Representative: **Priori, Enrico et al**
**Marks & Clerk France**
**Conseils en Propriété Industrielle**
**Immeuble «Visium»**
**22, avenue Aristide Briand**
**94117 Arcueil Cedex (FR)**

(56) References cited:
**WO-A1-99/07756**
**US-A1- 2008 021 129**
**US-A1- 2008 139 769**
**US-A1- 2012 053 313**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

The following specification particularly describes the invention and the manner in which it is to be performed:

## TECHNICAL FIELD OF INVENTION

The present invention relates to hydrophobic, flexible copolymer with high refractive index. Particularly, present invention relates to hydrophobic, flexible copolymer useful in ophthalmic lenses, particularly intraocular lens. More particularly, present invention relates to process for the preparation of hydrophobic, flexible copolymer.

## BACKGROUND OF THE INVENTION

The natural crystalline lens in the eye provides the ability of focusing objects placed at different distances in a process referred as accommodation. The crystalline lens grows throughout life increasing in size and rigidity. Due to this growth, the accommodation capability decreases with age (presbyopia) and an external optical correction is often needed to focus at objects located at near distances. At the same time, the lens becomes gradually opacified and results in loss in transparency termed as cataract formation.
Cataract is the most common cause of visual loss in the world. Aging or stress can change the morphology of the proteins, causing the natural lens to lose transparency. Cataract formation is irreversible and can eventually cause blindness. Cataract surgery is among the most common major surgical procedures performed on the elderly in developed countries. The technique consists of implanting an intraocular lens (IOL) after surgically removing the opacified or otherwise damaged natural crystalline lens.
The natural crystalline lens is a precisely formed structure consisting of 65% water and 35% organic material (mostly structural proteins). The proteins are structured in such a manner that there are negligible local variations in their density, resulting in a transparent material. The natural lens has a refractive index of 1.42.
Intraocular lens (IOL) implantation is performed after cataract removal to replace the optical function of the natural lens. The first material used for IOL implantation was poly(methyl methacrylate) (PMMA). PMMA has good optical properties and is compatible with the tissues of the eye, but the glass transition temperature of PMMA makes it rigid at room temperature.
To reduce the trauma to the eye in cataract surgery, it is desirable to keep the incision through which the surgical procedure is conducted as small as possible. The incision to insert the optic was about 6-7 mm. With the development of phacoemulsification, it became possible to liquefy the natural lens and remove through a 1.5 mm incision. However, using a room temperature foldable material, the incision to implant the IOL could be made 3-4 mm in diameter, requiring no sutures.
An increase of the refractive index is always desirable because it permits the manufacture of thinner IOL and subsequent reduction in size of the incision. The material used for opthalmic devices should be stable to prolonged exposure to UV light, natural wear and tear, should remain transparent and glistening free, vacuoles free over the extended period of time at the normal temperature and physiological conditions of the eye. As a result, various silicone, acrylate and hydrogel lenses have been developed for IOLs.
The use of hydrophobic polymers in IOL’s has been accepted by the medical fraternity for having good physical properties and acceptable bio compatibility in ocular region. A number of hydrophobic acrylate and methacrylate monomers are used for IOL fabrication since these are characterized by high refractive index (RI) allows for small incision during surgery and better transparency.
US5290892 disclose flexible intraocular lenses made from high refractive index polymers comprising a copolymer with an elongation of at least 200% comprised of monomers selected from aromatic acrylates or methacrylates, and a polymerisable cross-linking agent. The said patent further discloses ultraviolet absorbing material 2-(3’-methallyl -2-hydroxy-5’-methyl-phenyl) benzotriazole. The copolymer has a glass transition temperature of about 37°C and refractive index of about 1.552±.0.004.
In certain cases, foldable acrylic intraocular lenses develop "glistenings" when implanted in humans or soaked in water at physiological temperatures. Although glistenings have no detrimental effect on the function or performance of IOL’s made from acrylic materials, it is nevertheless cosmetically desirable to minimize or eliminate them. PCT publication No.WO9724382 relate to foldable intraocular lens material comprising copolymer of 2-phenylethylacrylate and 2-phenylethymiethacrylate and 0.1 to 10 mole% of a third monomer having hydrophilic characteristics which include monomers such as acrylic acid, methacrylic acid, hydroxyethylacrylate, hydroxyethylmethacrylate, acrylamide, methacrylamide, poly (ethylene glycol) acrylates (PEG-acrylates) and other similar monomers (preferably unsaturated compounds), especially those containing carboxyl-, hydroxyl-, sulphate, sulphonate-, amido-or substituted amino-bearing groups intended to minimize the risk for glistening.
Hydrophilic ingredients are undesirable as these materials reduce the overall refractive index of the hydrophobic

aryl acrylic material, since the hydrophilic material possess low refractive index.

There are many Patent/Patent Applications which disclose foldable co-polymeric compositions containing hydrophobic/hydrophilic acrylate and methacrylate monomers either alone or in combination for IOL, a few which are listed herein such as WO9907756, US2012196951, US2008139769, WO1994/011764, US7585900, US5331073, US5403901, US5674960, EP1030194, US6653422, US5693095, US5433746, US5693095, US2008/021129 and US2012/053313. The said Patent/Patent Applications suffer from drawbacks such as tackiness, opacity, low internal reflections, low tensile strength; some applications disclose the use of halogenated monomers to rectify the tackiness thereby adding to the cost and other undesirable problems which impact the use of IOL for longer period of time.

Further, IOL's must overcome issues such as cytotoxicity and biocompatibility that may arise due to extractible contaminants. To reduce these impurities extraction steps are carried out. Prior art includes WO2004/029675, US2005258096, US2004031275, which disclose batch extraction process.

The present inventors observed that intra ocular lens (IOL) designed to be in direct contact with the living tissue of the eye or its immediate surroundings in addition being biocompatible should have certain physical properties such as flexibility, low unfolding time, low glass transition temperature to avoid rigidity, and other characteristics that will enable its use for a longer period of time. They further observed that even incremental change in the refractive index can make substantial difference to the user suffering from failing eye sight and vision.

In view of the above, the present inventors contemplated that it would be advantageous to provide an improved hydrophobic co-polymeric material comprising of novel monomers that can be used for making thinner optical articles than conventional used ones, are flexible with high refractive index and are patient friendly.

It was further observed that the mechanical characteristics of the resulting copolymer material varies with the selection of monomer and its relative proportions, combinations of monomers with other constituents such as cross-linking entities and the conditions employed during polymerization such as the amount of initiator, delivery of activation energy (thermal or radiated; e.g. ultraviolet light) and other components. Additionally, other differences in the nature of the monomers, such as polarity and solubility, can prevent the polymerized uni- monomeric blocks from forming homogeneous blends possibly leading to differences in the physical properties of the resultant polymer.

## OBJECTIVES OF THE INVENTION

Main object of the present invention is to provide a copolymer which is hydrophobic, foldable and has high refractive index suited to use in ophthalmic lenses, particularly intraocular lens(IOLs).

Another object of the present invention is to provide a process for synthesis of the copolymer that has the desired improved functional properties such as high refractive index, low glass transition temperature ($T_g$), controlled unfolding rate and low water content for use in ophthalmic lenses, particularly IOL's.

## SUMMARY OF THE INVENTION

Accordingly present invention according to claims 1-5, 7-10 provides a copolymer comprising 5 to 95 mol % monomer of formula I;

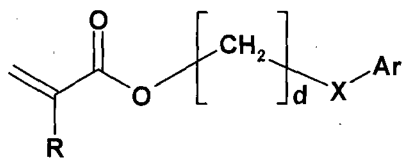

Formula I

wherein;

R is H or alkyl; 'd' is 1-10

X may be present or absent; with the proviso when X is present, X represents O, S or $NR^2$ where $R^2$ is H, (un)substituted or substituted alkyl, (un)substituted or substituted aryl, $CH_2C_6H_5$;

Ar represents an aromatic ring which is (un) substituted or substituted with H, $CH_3$, $CF_3$, $C_2H_5$, alkyl, $OCH_3$, $C_6H_{11}$, Cl, Br, $C_6H_5$, or $CH_2C_6$ $H_5$;

5 to 95 mol% monomer of formula II;

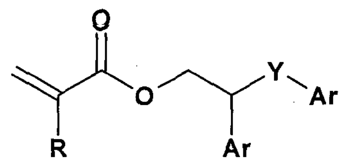

Formula II

wherein;

R is H or alkyl;

Y represents O or S;

Ar represent an aromatic ring which is (un) substituted or substituted with H, $CH_3$, $CF_3$, $C_2H_5$, alkyl, $OCH_3$, $C_6H_{11}$, Cl, Br, $C_6H_5$, or $CH_2C_6H_5$; and

and 0.1 to 20 mol% a co-polymerizable cross linker selected from the group consisting of terminally ethylenically unsaturated compounds such as hexanediol dimethacrylate (HDDMA), Ethylene glycol dimethacrylate (EGDMA), allyl methacrylate, diallyl methacrylate; pentaerythritol tetra(meth)acrylate, 1,3-propanediol dimethacrylate, 1,4-butanediol dimethacrylate; and their corresponding acrylates.

In an embodiment of the present invention, said copolymer comprising:

i. co-polymer of 2-phenylethyl acrylate (PEA), 2-phenoxy-2-phenylethyl acrylate (PPEA) and ethylene glycol dimethacrylate;
ii. co-polymer of 2-phenylethyl acrylate (PEA), 2-phenoxy-2-phenylethyl acrylate (PPEA) and hexanediol dimethacylate;
iii. co-polymer of 2-phenylethyl acrylate (PEA), 2-phenoxy-2-phenylethyl methacrylate (PPEM) and ethylene glycol dimethacrylate;
iv. co-polymer of 2-phenylethyl acrylate (PEA), 2-phenoxy-2-phenylethyl methacrylate (PPEM) and hexanediol dimethacylate;
v. co-polymer of 2-phenylethyl acrylate (PEA), 2-phenyl-(2-phenylthio)ethyl acrylate (PTEA) and ethylene glycol dimethacrylate;
vi. co-polymer of 2-phenylethyl acrylate (PEA), 2-phenyl-(2-phenylthio)ethyl acrylate (PTEA) and hexanediol dimethacylate;
vii. co-polymer of 2-phenylethyl acrylate (PEA), 2-phenyl-(2-phenylthio)ethyl methacrylate (PTEM) and ethylene glycol dimethacrylate; and
viii co-polymer of 2-phenylethyl acrylate (PEA), 2-phenyl-(2-phenylthio)ethyl methacrylate (PTEM) and hexanediol dimethacylate.

In another embodiment of the present invention, said copolymer is hydrophobic and exhibit refractive index in the range of 1.558 to 1.601.

In yet another embodiment of the present invention, tensile strength of the said copolymer is in the range of 1.58 to 12.69 MPa (of 230 to 1841 psi).

In yet another embodiment of the present invention, said copolymer is flexible exhibit modulus of elasticity in the range of 0.03 to 0.07 MPa (of 4 to 10 psi).

In yet another embodiment, present invention provides a process according to claim 6 for the preparation of hydrophobic copolymer of formula (I) as claimed in claim 1 by free radical copolymerization and the said process comprising the steps of:

i. mixing monomer I and monomer II in the ratio ranging between 5:95 to 95:5 mol% with cross linker and 0.1- 5.0 mol% radical initiator in centrifuge tube and vortexing to allow homogenization;
ii. passing nitrogen gas through the mixture followed by injecting the mixture into the glass moulds;
iii. heating the glass moulds at 60°C for 20 h followed by isothermal heating at 90 °C for 10 h until complete polymerization; and
iv. extracting the cross-linked polymer network using soxhlet extraction with 2-propanol to remove the unreacted monomers and oligomers until equilibrium.

In yet another embodiment of the present invention, the monomer of formula (I) is selected from 2-phenylethyl acrylate (PEA), 2-phenoxyethyl methacrylate; 2-phenoxyethyl acrylate, benzyl acrylate, 3-phenylpropyl acrylate, 4-phenylbutyl acrylate, 2-(phenylthio)ethyl actylate, 2-(phenylamino)ethyl acrylate.

In yet another embodiment of the present invention, the monomer of formula (II) is selected from 2-phenoxy-2-phenylethyl acrylate (PPEA) or 2-phenoxy-2-phenylethyl methacrylate (PPEM) or 2-phenyl-(2-phenylthio)ethyl acrylate (PTEA) or 2-phenyl-(2-phenylthio)ethyl methacrylate (PTEM).

In yet another embodiment of the present invention, the cross linker is in the range of 1-3 mol% w.r.t the mol % of the combination of monomers.

In yet another embodiment of the present invention, the co-polymer may optionally comprise additives selected from UV absorbers, dyes, light stabilizers, coating materials, pharmaceutical agents, cell receptor functional groups, viscosity agents, diluents or combinations thereof wherein the UV absorber used is in the range of 0.1 to 2.0 wt % of total monomers.

## DETAILED DESCRIPTION OF THE FIGURES

**Fig 1** depicts DSC thermograms of poly(PEA-co-PPEA) networks
**Fig 2** depicts Spectral transmittance of poly(PEA-co-PPEA) networks
**Fig 3** depicts Stress vs strain curves for poly(PEA-co-PPEA) networks
**Fig 4** depicts Cell viability of L929 mouse connective tissue fibroblasts as the result of MMT assay for representative poly(PEA-co-PPEA) networks with positive and negative controls.
**Fig 5** depicts TGA thermograms for representative poly (PEA-co-PPEA) networks
**Fig 6** depicts DSC thermograms of poly(PEA-co-PPEM) networks
**Fig 7** depicts Spectral transmittance of poly(PEA-co-PPEM) networks
**Fig 8** depicts Stress vs strain curves for poly(PEA-co-PPEM) networks
**Fig 9** depicts Cell viability of L929 mouse connective tissue fibroblasts as the result of MMT assay for representative poly(PEA-co-PPEM) networks with positive and negative controls.
**Fig 10** depicts TGA thermograms for representative poly (PEA-co-PPEM) networks
**Fig 11** depicts DSC thermograms of poly(PEA-co-PTEA) networks
**Fig 12** depicts Spectral transmittance of poly(PEA-co-PTEA) networks
**Fig 13** depicts Stess vs strain curves for poly(PEA-co-PTEA) networks
**Fig 14** depicts Cell viability of L929 mouse connective tissue fibroblasts as the result of MMT assay for representative poly(PEA-co-PTEA) networks with possitive and negative controls.
**Fig 15** depicts TGA thermograms for representative poly(PEA-co-PTEA) networks
**Fig 16** depicts DSC thermograms of poly(PEA-co-PTEM) networks
**Fig 17** depicts Spectral transmittance of poly(PEA-co-PTEM) networks
**Fig 18** depicts Stess vs strain curves for poly(PEA-co-PTEM) networks
**Fig 19** depicts Cell viability of L929 mouse connective tissue fibroblasts as the result of MMT assay for representative poly(PEA-co-PTEM) networks with possitive and negative controls.
**Fig 20** depicts TGA thermograms for representative poly(PEA-co-PTEM) networks.

Scheme 1 represents synthesis of poly (PEA-co-PPEA) bulk polymer networks wherein the variable 's' in the cross linker is 1 or 3.

Scheme 2 represents synthesis of poly(PEA-*co*-PPEM) bulk polymer networks wherein the variable 's' in the cross linker is 1 or 3.

Scheme 3 represents synthesis of poly(PEA-co-PTEA) bulk polymer networks; wherein the variable 's' in the cross linker is 1 or 3)

Scheme 4 represents synthesis of poly(PEA-co-PTEM) bulk polymer networks; wherein the variable 's' in the cross linker is 1 or 3.

## DETAILED DESCRIPTION OF THE INVENTION

The present invention relates to hydrophobic, flexible copolymer with high refractive index copolymer comprising a mixture of a monomer of formula I;

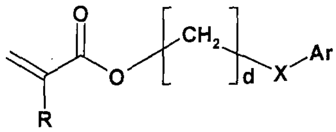

Formula I

wherein;

R is H or alkyl; 'd' is 1-10
X may be present or absent; with the proviso when X is present, X represents O, S or $NR^2$ wherein $R^2$ is H, (un)substituted or substituted alkyl, (un)substituted or substituted aryl, $CH_2C_6H_5$;
Ar represents an aromatic ring which can be (un)substituted or substituted with H, $CH_3$, $CF_3$, $C_2H_5$, alkyl, $OCH_3$, $C_6H_{11}$, Cl, Br, $C_6H_5$, or $CH_2C_6$ $H_5$;

a monomer of formula II;

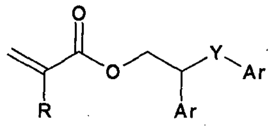

Formula II

wherein;

R is H or alkyl;
Y represents O or S;

Ar represents an aromatic ring which can be (un)substituted or substituted with H, $CH_3$, $CF_3$, $C_2H_5$, alkyl, $OCH_3$, $C_6$ $H_{11}$, Cl, Br, $C_6$ $H_5$, or $CH_2C_6$ $H_5$; and
a co-polymerizable cross linker selected from the group consisting of terminally ethylenically unsaturated compounds such as hexanediol dimethacrylate (HDDMA), ethylene glycol dimethacrylate (EGDMA), allyl methacrylate, diallyl methacrylate; pentaerythritol tetra(meth)acrylate, 1,3-propanediol dimethacrylate, 1,4-butanediol dimethacrylate; and their corresponding acrylates.

The monomers (I) include but are not limited to 2-phenylethyl acrylate (PEA), 2-phenoxyethyl methacrylate; 2-phenoxyethyl acrylate; phenyl acrylate;; benzyl acrylate, 3-phenylpropyl acrylate; 4-phenylbutyl acrylate; 2-(phenylthio)ethyl actylate; 2-(phenylamino)ethyl acrylate.

The monomer (II) include but are not limited to 2-phenoxy-2-phenylethyl acrylate (PPEA) or 2-phenoxy-2-phenylethyl methacrylate (PPEM) or 2-phenyl-(2-phenylthio)ethyl acrylate (PTEA) or 2-phenyl-(2-phenylthio)ethyl methacrylate (PTEM).

The copolymer of the instant invention may optionally comprise additives selected from UV absorbers, dyes, light stabilizers, coating materials, pharmaceutical agents, cell receptor functional groups, viscosity agents, diluents or combinations thereof. The ultraviolet absorbing material can be any compound which absorbs ultraviolet light having a wavelength shorter than about 400 nm, but does not absorb any substantial amount of visible light. The ultraviolet absorbing compound is incorporated into the monomer mixture and is entrapped in the polymer matrix when the monomer mixture is polymerized. The ultraviolet absorbing compounds are selected from the compound which are co-polymerizable with the monomers and thereby is covalently bound to the polymer matrix thereby minimizing the leaching of the ultraviolet absorbing compound out of the lens and into the interior of the eye is minimized. The co-polymerizable ultraviolet absorbing compounds are the substituted 2-hydroxybenzophenones disclosed in US 4,304,895; the 2-hydroxy-5-acryloxyphenyl-2H-benzotriazole disclosed in US 4,528,311 and 5-chloro-2-[2-hydroxy-5-(3-methacryloyloxyethylcarbamoyloxyethyl)] phenyl-2H-benzotriazole and 2-[2-hydroxy-5-(δ-methacryloyloxyethyl-carbamoyloxyethyl)] phenyl-2H-benzotriazole disclosed in U.S. patent No. 5814680. The most preferred ultraviolet absorbing compound is 2-(2'-hydroxy-3'-methallyl-5'-methyl phenyl) benzotriazole. The uv absorber may be used in the range of 0.1 to 2.0 wt% w.r.t the mol % of the monomers.

The present invention relates to free radical copolymerization of the monomers in the presence of cross-linker and radical initiator to obtain hydrophobic, flexible copolymer with high refractive index useful in foldable IOLs.

The free radical polymerization is usually carried out in the presence of monofunctional initiator. The initiator concentration and polymerization temperature are primary factors regulating the polymerization rate and molecular

weight of the product. In the process of the present invention, radical initiator such as 2,5-dimethyl-2,5-di(2-ethylhexanoylperoxy)hexane (Luperox-256), is used to initiate bulk free radical polymerization.

The process for synthesis of flexible, high refractive index copolymer useful in foldable IOLs by radical copolymerization includes the following steps;

i. mixing monomer (I), monomer (II), cross linker and a radical initiator in centrifuge tube and vortexing to allow homogenization;

ii. passing nitrogen gas through the mixture followed by injecting the mixture into the glass moulds;

iii. heating the glass moulds at 60°C for 20 h followed by isothermal heating at 90 °C for 10 h until complete polymerization; and

iv. extracting the cross-linked polymer network using soxhlet extraction with 2-propanol to remove the unreacted monomers and oligomers until equilibrium is reached.

According to the process, monomer mixtures, cross linker and radical initiator are weighed in centrifuge tubes and vortexed to allow homogenization. Nitrogen gas is bubbled through the mixture to remove most of the oxygen present. The mixtures are syringed into glass moulds. Glass plates of the mould are separated by square shaped PTFE spacer of 0.5 mm thickness. The glass moulds are heated at 60 °C for 20 h followed by isothermal heating at 90 °C for 10 h to ensure complete polymerization. After polymerization, the cross-linked polymer networks are extracted using soxhlet extraction with 2-propanol to remove the unreacted monomers and oligomers until equilibrium is reached.

The monomers and its relative proportion is one of the key factors to affect the characteristics of the resulting copolymer and fabrication into IOLs. Depending on the combination, mechanical characteristics of the resulting material can affect ease of folding and subsequent unfolding time, stability of the lens after implantation; and sensitivity to changes in temperature. Further, the structure of a particular monomer is observed to affect the magnitude of the polymer's $T_g$ (glass transition temperature).

The monomer (I), monomer (II) and cross linker used in the process are as described hereinabove. The monomer composition in the copolymerization process varies in the range of 10:40 to 90:60 mol%. The cross linker is used in the range of 1-3mol% w.r.t the mol % of the combination of monomers. The concentration of radical initiator is in the range 0.1-0.3 mol%.

In another preferred embodiment, the copolymer network of the present invention comprises:

i. co-polymer of 2-phenylethyl acrylate (PEA), 2-phenoxy-2-phenylethyl acrylate (PPEA) and ethylene glycol dimethacrylate;

ii. co-polymer of 2-phenylethyl acrylate (PEA), 2-phenoxy-2-phenylethyl acrylate (PPEA) and hexanediol dimethacylate;.

iii. co-polymer of 2-phenylethyl acrylate (PEA), 2-phenoxy-2-phenylethyl methacrylate (PPEM) and ethylene glycol dimethacrylate;

iv. co-polymer of 2-phenylethyl acrylate (PEA), 2-phenoxy-2-phenylethyl methacrylate (PPEM) and hexanediol dimethacylate;

v. co-polymer of 2-phenylethyl acrylate (PEA), 2-phenyl-(2-phenylthio)ethyl acrylate (PTEA) and ethylene glycol dimethacrylate;

vi. co-polymer of 2-phenylethyl acrylate (PEA), 2-phenyl-(2-phenylthio)ethyl acrylate (PTEA) and hexanediol dimethacylate;

vii. co-polymer of 2-phenylethyl acrylate (PEA), 2-phenyl-(2-phenylthio)ethyl methacrylate (PTEM) and ethylene glycol dimethacrylate; and

viii. co-polymer of 2-phenylethyl acrylate (PEA), 2-phenyl-(2-phenylthio)ethyl methacrylate (PTEM) and hexanediol dimethacylate.

The present invention discloses synthesis of copolymer networks of 2-phenyl ethyl acrylate (PEA), 2-phenoxy-2-phenylethyl acrylate (PPEA) by free radical copolymerization comprising;

i. mixing 2-phenylethyl acrylate (PEA) and 2-phenoxy-2-phenylethyl acrylate (PPEA) in presence of 2 mol% of ethylene glycol dimethacrylate or hexanediol dimethacylate as a cross-linker and 0.2 mol% 2,5-dimethyl-2,5-di(2-ethylhexanoylperoxy)hexane in centrifuge tube and vortexing to allow homogenization;

ii. passing nitrogen gas through the mixture followed by injecting the mixture into the glass moulds; ,

iii. heating the glass moulds at 60°C for 20 h followed by isothermal heating at, 90°C for 10 h until complete polymerization;

iv. extracting the cross-linked poly(PEA-co-PPEA) networks using soxhlet extraction with 2-propanol to remove the unreacted monomers and oligomers until equilibrium is reached.

The co-polymerization process is given in **Scheme 1.**

The monomers are added in varying molar ratios and are given in **Table 1** below in examples.

The present invention discloses synthesis of copolymer networks of 2-phenylethyl acrylate (PEA) and 2-phenoxy-2-phenylethyl methacrylate (PPEM) comprising;

i. mixing 2-phenylethyl acrylate (PEA) and 2-phenoxy-2-phenylethyl methacrylate (PPEM) in presence of 2 mol % of ethylene glycol dimethacrylate or hexanediol dimethacylate as cross-linker and 0.2 mol% 2,5-dimethyl-2,5-di(2-ethylhexanoylperoxy)hexane in centrifuge tube and vortexing to allow homogenization;
ii. passing nitrogen gas through the mixture followed by injecting the mixture into the glass moulds;
iii. heating the glass moulds at 60°C for 20 h followed by isothermal heating at 90 °C for 10 h until complete polymerization; and
iv. extracting the cross-linked poly(PEA-co-PPEA) network using soxhlet extraction with 2-propanol to remove the unreacted monomers and oligomers until equilibrium is reached. The copolymerization process is given in **Scheme 2.**

The monomers are added in varying molar ratios and are given in **Table 4** below.

In yet another embodiment, the present invention discloses synthesis of copolymer networks of 2-phenylethyl acrylate (PEA) and 2-phenyl-(2-phenylthio) ethyl acrylate (PTEA) comprising;

i. mixing 2-phenylethyl acrylate (PEA) and 2-phenyl-(2-phenylthio)ethyl acrylate (PTEA) in presence of 2 mol % of ethylene glycol dimethacrylate or hexanediol dimethacylate as cross-linker and 0.2 mol% 2,5-dimethyl-2,5-di(2-ethylhexanoylperoxy)hexane in centrifuge tube and vortexing to allow homogenization;
ii. passing nitrogen gas through the mixture followed by injecting the mixture into the glass moulds;
iii. heating the glass moulds at 60°C for 20 h followed by isothermal heating at 90°C for 10 h until complete polymerization; and
iv. extracting the cross-linked poly(PEA-co-PPEA) with cross linker and uv absorber network using soxhlet extraction with 2-propanol to remove the unreacted monomers and oligomers until equilibrium is reached. The copolymerization process is given in **Scheme 3.**

The monomers are added in varying molar ratios and are given in **Table 7** below.

The present invention discloses synthesis of copolymer networks of 2-phenylethyl acrylate (PEA) and 2-phenyl-(2-phenylthio)ethyl methacrylate (PTEM) comprising;

i. mixing 2-phenylethyl acrylate (PEA) and 2-phenyl-(2-phenylthio)ethyl methacrylate (PTEM) in presence of 2 mol % of ethylene glycol dimethacrylate or hexanediol dimethacylate as cross-linker and 0.2 mol% 2,5-dimethyl-2,5-di(2-ethylhexanoylperoxy)hexane in centrifuge tube and vortexed to allow homogenization;
ii. passing nitrogen gas through the mixture followed by injecting the mixture into the glass moulds;
iii. heating the glass moulds at 60°C for 20 h followed by isothermal heating at 90 °C for 10 h until complete polymerization; and
iv. extracting the cross-linked poly(PEA-co-PTEM) with cross linker and uv absorber networks using soxhlet extraction with 2-propanol to remove the unreacted monomers and oligomers until equilibrium is reached. The copolymerization process is given in Scheme 4.

The monomers are added in varying molar ratios and are given in **Table 10** below. The free radical copolymerization process of the current invention using monomer of formula (I), monomer of formula (II), cross linkers in presence of radical initiator 2,5-dimethyl-2,5-di(2-ethylhexanoylperoxy)hexane affords in the synthesis of copolymer with desired properties such as low glass transition temperature (5 to 20°C), high refractive index (1.55+), controlled unfolding rate (5 to 60 sec), and low equilibrium water content (< 1wt%) which are useful for fabricating foldable ophthalmic lenses preferably IOLs.

## Experimental

### 1.1 Materials

All monomers were purified by column chromatography before use. Ethylene dimethacrylate (EGDMA) and hexanediol dimethacrylate (HDDMA) were procured from Sigma Aldrich. 2,5-dimethyl-2,5-di(2-ethylhexanoylperoxy)hexane (Luperox-256), the thermal initiator, was procured from Arkema Inc. and used as received. 2-propanol (Merck) was used as received.

### 1.2 Polymerization process

Polymer networks were prepared by radical co-polymerization of monomer (I), monomer (II) in the presence of cross-linker and radical initiator. Monomer mixtures (formula I and II) were weighed in centrifuge tubes and vortexed to allow the homogenization. Nitrogen gas was bubbled through the mixture to remove most of the oxygen present. The mixtures were then syringed into glass moulds. Glass plates of the mould were separated by square shaped PTFE spacer of 0.5 mm thickness. The glass moulds were heated at 60 °C for 20 h followed by isothermally heating at 90 °C for 10 h to ensure complete polymerization. After polymerization, the cross-linked polymer networks were extracted using soxhlet extraction with 2-propanol to remove the unreacted monomers and oligomers until equilibrium was reached.

### 1.3 Characterization techniques

#### 1.3.1 IR spectroscopy

The IR spectra of monomers and the prepared samples were taken using a Fourier transform infrared (FT-IR) spectrophotometer (Perkin-Elmer) between 400 and 4000 $cm^{-1}$.

#### 1.3.2 Differential scanning calorimetry (DSC)

Differential scanning calorimetry was performed on a TA Q100 DSC instrument to determine the glass transition temperatures ($T_g$) of the copolymers. All samples were run against an aluminium reference in crimped aluminium pans. A temperature range of -50 to 120 °C was used to determine the $T_g$. Two scans were performed on each sample at a heating rate of 10 °C/min. The second heating results were obtained in all cases.

#### 1.3.3 Refractometry (R.I.)

Refractive index measurements were performed with an automatic refractometer, model PTR 46 X, from Index Instrument and calibrated with a standard. Each sample was allowed to equilibrate to 20 °C prior to the measurements. The measurements were done in triplicate and the average values recorded.

#### 1.3.4 Transparency

The transparency of the polymer networks was measured with a Lambda-35 Perkin Elmer Ultraviolet-Visible spectrometer between 200 to 800 nm.

#### 1.3.5 Monomer/Oligomer extraction

In bulk polymerization, there will always be residual monomers at the end because a polymerization reaction does not proceed to the point where exactly 100% of the monomer is converted. Residual monomers in the polymer film are a problem for two reasons. First, any low molecular mass substances present in the polymer network, wherein the residual monomer, acts as a plasticizer. The rheological properties of the material will thus be different as compared to the same material without any residual monomers. Second, for medical applications like an IOL, a residual content of low molecular mass substances is potentially harmful because it will be released from the implanted IOL by diffusion. The residual monomer content of the cross-linked polymer networks made by chamber polymerization was determined.

Unreacted monomer and oligomer were removed by using soxhlet extraction. Each sample was placed between two cellulose extraction thimbles. The samples were weighed and the values recorded. The thimbles containing the sample were placed into a soxhlet extractor and the extractor placed on a 500 mL round bottom flask containing 250 mL 2-propanol. The 2-propanol was refluxed and the samples were extracted for 24 hours. Samples were then removed and dried in a vacuum oven at 65°C for eight hours. Samples were weighed. The process was repeated until no further weight loss was evident; usually 24 hours.

#### 1.3.6 Equilibrium water content (EWC)

Approximately 1g sample was cut from each polymer sheet and weighed dry. Samples were then placed in balanced salt solution (BSS) and allowed to hydrate for 24 hours. They were removed from the BSS, wiped dry, and the weights measured. The samples were rehydrated and measured again at five days. EWC was measured by the following equation 1:

$$EWC(wt\%) = \left[\frac{W_{wet} - W_{dry}}{W_{dry}}\right] \times 100 \qquad 1$$

where $W_{wet}$ is the weight of wet specimen at equilibrium and $W_{dry}$ is the initial weight of the dry specimen. In all the experiments a minimum of three samples were measured and averaged. The samples were allowed to hydrate until no further water uptake was observed.

Balanced salt solution (BSS) is a solution of sodium chloride (NaCl), potassium chloride (KCl), calcium chloride ($CaCl_2 \cdot 6H_2O$), magnesium chloride ($MgCl_2 \cdot 6H_2O$), sodium acetate ($C_2H_3NaO_2 \cdot 3H_2O$), and sodium citrate dihydrate ($C_6H_5Na_3O_7 \cdot 2H_2O$). BSS is isotonic to the tissues of the eyes.

### 1.3.7 Surface wettability (Water contact angle)

The contact angle measurement was performed to obtain the hydrophilicity /hydrophobicity of the lens materials. The basis of the contact angle measurement lies in the fact that the spreading of a drop on a surface is related to physical-chemical forces between the liquid and the material. The sessile-drop method in air was used to quantify wetting ability, an indicator of hydrophilicity. The contact angle reading taken is the angle between the bordering surfaces, in this case formed between the IOL and the water surface. The wetting ability (and therefore hydrophilicity) is inversely proportional to the contact angle and to surface tension.

Water contact angles (CAs) of the copolymer networks were measured using the sessile drop technique in a contact angle measuring system Easy Drop (Kruss) using water as contact liquid at ambient humidity and temperature. Drops of deionized water ~ 1.0 μL in volume were applied to the sample surface. A minimum of 10 drops were applied on each sample.

### 1.3.8 Unfolding rate analysis

Three disk shaped samples of 10 mm in diameter and 0.5 mm thickness were cut from each polymer sheet. The sample was folded in half with a pair of forceps and placed on a horizontal surface to unfold at 37 °C. The amount of time for the polymer disc to return to its original shape was recorded. Each measurement was done three times and the average was taken.

### 1.3.9 Mechanical tensile testing

Mechanical tensile testing was performed on standard dumbbell-shaped specimens cut from the prepared 0.5 mm thick polymer sheets by a dumbbell-shaped cutting knife. A Linkam Tensile Stress Testing System - TST 350 was employed to measure the elastic modulus, failure strain, as well as the tensile strength of the networks with a constant extension rate of 1.0 mm/sec at 37 °C.

### 1.3.10 Thermogravimetric analysis (TGA)

The thermal stability of the copolymers was studied by thermogravimetric analysis (TGA) employing a STA 6000 TGA model from Perkin Elmer instruments. The samples were heated at a rate of 10°C/min under nitrogen atmosphere.

### 1.3.11 Cytotoxicity

Biocompatibility was an important influencing factor for the application of copolymers in IOLs. Non-biocompatibility of IOLs could result in inflammatory response and posterior capsular opacification. In order to test whether the prepared copolymers were cytocompatible or cytotoxic, cyto compatibility tests were performed with L929 mouse fibroblasts according to ISO protocol 10993-5.

The mouse connective tissue fibroblast cell line L929 (National Centre for Cell Science, Pune, India) was adopted and cultured in Dulbecco's modified Eagle's medium (DMEM) containing 10% fetal bovine serum (FBS), 100 U/mL penicillin, and 100 μg/mL streptomycin at 37 °C under a humidified atmosphere of 5% $CO_2$. For subculture, the cell monolayer was washed twice with phosphate-buffered saline (PBS) and incubated with trypsin-EDTA solution (0.05% trypsin, 0.25% EDTA) for 5 min at 37 °C to detach the cells. Cells were re-suspended in culture medium and co-cultured with the prepared copolymer networks in DMEM supplemented with 10% FBS for 7 days in a 24-well plate. The prepared disc of copolymer networks (10 mm diameter) were washed with ethanol in an ultrasonic cleaner and sterilized by

autoclaving. The medium was changed every 2 to 3 days. Cultures were evaluated for cell viability/proliferation at days 1, 2, 3, 5 and 7 (MTT assay). Cells were trypsinised, washed with phosphate-buffer saline, centrifuged at 5000 rpm, fixed on a glass slide and observed by Carl Zeiss Axio scope microscope to assess cell morphology. High density polyethylene (HDPE) and organo-tin poly (vinyl chloride) (PVC) films were used as negative and positive controls for cytotoxicity testing.

## EXAMPLE

The following examples are given by way of illustration and therefore should not be construed to limit the scope of the present invention in any way.

### Example 1: Synthesis of copolymer networks of 2-phenylethyl acrylate (PEA) and 2-phenoxy-2-phenylethyl acrylate (PPEA)

Poly(PEA-co-PPEA) networks were prepared by radical co-polymerization of 2-phenylethyl acrylate (PEA), 2-phenoxy-2-phenylethyl acrylate (PPEA), in the presence of 2 mol % ethylene dimethacrylate (EGDMA) or hexanediol dimethacrylate (HDDMA) as a cross-linker and 0.2 mol% Luperox-256 as an initiator. The networks were prepared with varying molar ratios of PEA and PPEA. Monomers used and copolymerization feed compositions are detailed in **Table 1.** Around 4 g monomer mixtures were weighed in centrifuge tubes and the appropriate amount of each component was added. Mixtures were vortexed for 60 seconds to allow the homogenization. Nitrogen gas was bubbled through the mixtures to remove most of the oxygen present. Mixtures were then syringed into a glass molds. The glass molds were placed in an oven at 60°C for 20h followed by 90°C for 10h to ensure complete polymerization. After polymerization, the cross-linked poly(PEA-*co*-PPEA) networks were extracted using soxhlet extraction with 2-propanol to remove unreacted monomers and oligomers until equilibrium was reached.

**Table 1: Monomer compositions for PEA-PPEA bulk polymerisation system**

| Sr. No. | Code | PEA (mol %) | PPEA (mol %) | Cross-linker | % Wt. loss after solvent extraction |
|---|---|---|---|---|---|
| 1 | S1-EG-10 | 88.2 | 9.8 | 2 mol % EGDMA | 1.2 |
| 2 | S1-EG-15 | 83.3 | 14.7 | 2 mol % EGDMA | 1.0 |
| 3 | S1-EG-20 | 78.4 | 19.6 | 2 mol % EGDMA | 0.9 |
| 4 | S1-EG-25 | 73.5 | 24.5 | 2 mol % EGDMA | 1.3 |
| 5 | S1-EG-30 | 68.6 | 29.4 | 2 mol % EGDMA | 1.1 |
| 6 | S1-EG-35 | 63.7 | 34.3 | 2 mol % EGDMA | 1.4 |
| 7 | S1-EG-40 | 58.8 | 39.2 | 2 mol % EGDMA | 1.2 |
| 8 | S1-HD-20 | 78.4 | 19.6 | 2 mol % HDDMA | 1.0 |
| 9 | S1-HD-30 | 68.6 | 29.4 | 2 mol % HDDMA | 0.9 |

### 1.1 IR spectroscopy

Comparison of spectra of the monomers with that of representative copolymer networks showed absorption peak of C=C double bond at 1637 $cm^{-1}$ can be clearly observed in the monomers but disappeared in the copolymer. This indicated that the C=C double bond was almost reacted during the polymerization.

### 1.2 Differential scanning calorimetry (DSC)

Polymers based on (meth) acrylate monomers have potential for a broad range of thermo-mechanical properties, making them strong candidates for optical materials. As represented in **Figure1,** with the increase of the concentration of PPEA monomer from 10 mol% to 40 mol%, the $T_g$ of the copolymers increased from 5 to 18 °C. Copolymer networks with exorbitant $T_g$ are not suitable for biomedical applications. In the subsequent study, to achieve an appropriate $T_g$ of the prepared networks, the concentration of PPEA was determined to be 10 to 30 mol %.

**Table 2: Refractive index, glass transition temperature, water contact angle and unfolding time of poly(PEA-*co*-PPEA) networks**

| Sr. No. | Code | R.I. (at 589 nm) | $T_g$ (°C) | Contact Angle | Unfolding Time (sec) |
|---|---|---|---|---|---|
| 1 | S1-EG-10 | 1.560 | 3.53 | 76.01 | 4 |
| 2 | S1-EG-15 | 1.562 | 5.03 | 78.01 | 4 |
| 3 | S1-EG-20 | 1.564 | 6.84 | 79.80 | 6 |
| 4 | S1-EG-25 | 1.566 | 11.45 | 80.90 | 8 |
| 5 | S1-EG-30 | 1.568 | 14.79 | 81.31 | 11 |
| 6 | S1-EG-35 | 1.570 | 15.75 | 81.87 | 16 |
| 7 | S1-EG-40 | 1.572 | 21.49 | 82.16 | - |
| 8 | S1-HD-20 | 1.563 | 8.73 | 79.28 | 7 |
| 9 | S1-HD-30 | 1.566 | 16.89 | 82.91 | 14 |

**1.3 Refractometry (R.I.)**

**Table 2** shows the values of the Refractive Indices of poly(PEA-*co*-PPEA) networks. RI of copolymers increases from 1.560 to 1.571 with the increase in the concentration of PPEA.

**1.4 Transmittance**

The spectral transmittance of the poly(PEA-*co*-PPEA) networks is represented in **Figure 2.** Most of the copolymer networks showed excellent optical transparency, and their spectral transmittance was higher than 85% in the visible wavelength region of 400-800 nm.

**1.5 Equilibrium water content (EWC)**

The EWC of the all copolymers ranged from 0.1%-0.3%. These materials are hydrophobic in nature, so low EWC values were expected. The EWC values were measured on day 1 and day 5 after immersion into Balanced Salt Solution. No increase in EWC was seen after day 1.

**1.6 Water contact angle**

Water contact angles of the prepared copolymer networks, presented in **Table 2,** ranges from 76.01 to 82.91°. CA values indicated that hydrophobicity increased with the PPEA content in copolymer, due to higher aromatic character. The water drop profiles on the three representative samples S1-EG-10, 20, and 30 with their CA values were observed comparable to those marketed hydrophobic acrylic IOLs, whose CA generally ranges from 65 to 85°.

**1.7 Unfolding rate analysis**

The acceptable values for IOL unfolding times are 5 to 60 sec. The main concern is the IOL optic will unfold too rapidly and rupture the posterior capsule, creating unnecessary complications. For this reason, extremely long and short unfolding times are not favorable. Unfolding time for synthesized bulk polymer networks are presented in **Table 2.** The times measured were within the acceptable range. The S1-EG-10 composition tended to adhere to itself for a brief moment before unfolding. Sample S1-EG-40 was brittle and difficult to fold.

**1.8 Mechanical tensile testing**

**Figure 3** shows the stress plotted against its respective % strain determined from the mechanical tensile test. The tensile behavior was measured at 37 °C, which is beyond the $T_g$ of the networks. **Table 3** represents values of Young's Modulus at 100% strain, strength at failure strain and % elongation of synthesized polymer networks. The failure strain decreases as the elastic modulus increases, whereas the tensile strength increases as the elastic modulus increases. In the ophthalmic application, high failure strain is more important than high tensile strength. This is because

large deformation of the network is required to achieve a small incision size, whereas the stress generated from the eyeball is minimal. As a result, polymer networks with low elastic modulus and relatively high failure strain are very suitable for IOL.

**Table 3: Mechanical tensile testing parameters for poly(PEA-*co*-PPEA) networks**

| Code | Young's Modulus at 100 % strain (MPa) | Strength at failure strain (MPa) | % Elongation |
|---|---|---|---|
| S1-EG-10 | 0.003 | 1.27 | 190 |
| S1-EG-15 | 0.005 | 2.00 | 196 |
| S1-EG-20 | 0.004 | 3.74 | 280 |
| S1-EG-25 | 0.005 | 3.79 | 214 |
| S1-EG-30 | 0.005 | 5.14 | 270 |
| S1-EG-35 | 0.002 | 6.41 | 237 |
| S1-EG-40 | 0.008 | 8.41 | 171 |
| S1-HD-20 | 0.007 | 3.17 | 168 |
| S1-HD-30 | 0.007 | 4.97 | 158 |

### 3.9 Cytotoxicity

Cytotoxicity of the prepared polymer networks to L929 mouse fibroblasts was tested. L929 mouse fibroblasts were cultured for 7 days in direct contact to the networks. The effects of the networks on cell morphology were investigated by Carl Zeiss Axio scope microscope. A comparison of the microscopy images taken in bright field at 10X magnification obtained after incubation with polymer networks with the negative control and positive controls showed that Live L929 mouse fibroblast adherent cells can propagate to a confluent monolayer with increase in the culture time, as could be seen from those of negative control. The death of the cell was observed using a positive control, the open area between cells indicated that cell lysis had occurred. In contrast, the fibroblast L929 cells incubated with polymer networks maintained their morphology typical of L929. No cell debris and no detachment from dish bottom was observed. Results regarding cell viability (MTT assay) of cultures are shown in **Fig 4.** L929 cells presented a high proliferation rate throughout the culture time. At early incubation times, that is, day 1, values of MTT reduction were similar in seeded networks and negative controls, suggesting an identical number of attached cells, whereas the cell viability of the positive control went down to ~19 % in one day and almost to 0 % in two days. On day 7, the cell viability was stable (almost 100 %) for the negative control, whereas for the networks, it was a bit lower than that of the negative control and much higher than that of the positive control. These results suggested a lack of Cytotoxicity of polymer networks developed in this study, which was critical for their biomedical application in the reduction of inflammatory response and PCO.

### 3.10 Thermogravimetric analysis (TGA)

The thermal degradation of copolymers were characterised using TGA under nitrogen atmosphere. The TGA curves **(Fig 5)** clearly indicates that all polymers undergo single step degradation. In all polymers the weight loss was found to occur in a single step, starting at ~300 °C.

### Example 2: Synthesis of copolymer networks of 2-phenylethyl acrylate (PEA) and 2-phenoxy-2-phenylethyl methacrylate (PPEM).

Poly(PEA-*co*-PPEM) networks were prepared by radical co-polymerization of 2-phenylethyl acrylate (PEA) and 2-phenoxy-2-phenylethyl methacrylate (PPEM) in the presence of 2 mol % ethylene dimethacrylate (EGDMA) or hexanediol dimethacrylate (HDDMA) as a cross-linker, and 0.2 mol% Luperox-256 as an initiator. The networks were prepared with varying molar ratio of PEA and PPEM. Monomers used and co-polymerization feed compositions are detailed in **Table 4.** Around 5 g monomer mixtures were weighed in centrifuge tubes and the appropriate amount of each component was added. Mixtures were vortexed for 60 seconds to allow the homogenization. Nitrogen gas was bubbled through the mixtures to remove most of the oxygen present. Mixtures were then syringed into glass molds. The glass molds were placed in an oven at 60 °C for 20 h followed by 90 °C for 10 h to ensure complete polymerization. After polymerization, the cross-linked poly(PEA-*co*-PPEM) networks were extracted using soxhlet extraction with 2-propanol to remove unreacted monomers and oligomers until equilibrium was reached.

**Table 4: Monomer compositions for PEA-PPEM bulk polymerisation system**

| Sr. No. | Code | PEA (mol %) | PPEM (mol %) | Cross-linker | % Wt. loss after solvent extraction |
|---|---|---|---|---|---|
| 1 | S2-EG-10 | 88.2 | 9.8 | 2 mol % EGDMA | 1.1 |
| 2 | S2-EG-15 | 83.3 | 14.7 | 2 mol % EGDMA | 0.9 |
| 3 | S2-EG-20 | 78.4 | 19.6 | 2 mol % EGDMA | 1.1 |
| 4 | S2-EG-25 | 73.5 | 24.5 | 2 mol % EGDMA | 1.2 |
| 5 | S2-EG-30 | 68.6 | 29.4 | 2 mol % EGDMA | 1.0 |
| 6 | S2-EG-40 | 58.8 | 39.2 | 2 mol % EGDMA | 1.3 |
| 7 | S2-HD-20 | 78.4 | 19.6 | 2 mol % HDDMA | 0.8 |
| 8 | S2-HD-30 | 68.6 | 29.4 | 2 mol % HDDMA | 0.9 |

**2.1 IR spectroscopy**

The FTIR spectra of two representative copolymers with different cross-linkers, as well as the corresponding monomers shows that absorption peak of C=C double bond at 1637 $cm^{-1}$ can be observed clearly in the monomers, but disappears in the copolymers, which indicates that the C=C double bonds were almost exhausted during the polymerization.

**2.2 Differential scanning calorimetry (DSC)**

Generally, the $T_g$ increased as the rigidity in the molecule increased or by the addition of an *a*-methyl group. As shown in **Figure 6** and **Table 5,** with the increase of the concentration of PPEA monomer from 10 mol% to 40 mol%, the $T_g$ of the co-polymers increased from 1 to 21 °C. This is due to the fact that the asymmetrically substitution of the methyl group on the quaternary carbon in the main chain and aromatic rings in pendent group increases the steric hindrance and the internal rotation of the molecular chain is hindered. Copolymer networks with exorbitant $T_g$ were not suitable for biomedical applications. In the subsequent study, to achieve an appropriate $T_g$ (20 °C) of the prepared networks, the concentration of PPEM was determined to be 10 to 30 mol %. Polymer $T_g$ values higher than 20 °C were not suitable due to the rigid nature of the material at operating room temperatures.

**Table 5: Refractive index, glass transition temperature, water contact angle and unfolding time of poly(PEA-*co*-PPEM) networks**

| No. | Sr. Code | R.I. (at 589 nm) | $T_g$ (°C) | Contact Angle | Unfolding Time (sec) |
|---|---|---|---|---|---|
| 1 | S2-EG-10 | 1.558 | 1.53 | 76.86 | 3 |
| 2 | S2-EG-15 | 1.561 | 2.82 | 78.26 | 5 |
| 3 | S2-EG-20 | 1.563 | 8.42 | 81.74 | 8 |
| 4 | S2-EG-25 | 1.566 | 13.52 | 82.53 | 10 |
| 5 | S2-EG-30 | 1.567 | 18.70 | 83.03 | 16 |
| 6 | S2-EG-40 | 1.572 | 21.21 | 85.01 | - |
| 7 | S2-HD-20 | 1.562 | 11.18 | 79.32 | 7 |
| 8 | S2-HD-30 | 1.566 | 19.59 | 83.65 | 17 |

**2.3 Refractometry (R.I.)**

**Table 5** represents the values of the refractive indices of poly(PEA-*co*-PPEM) networks. RI of copolymers increases from 1.558 to 1.572 with the increase in the concentration of PPEM.

### 2.4 Transmittance

After the polymerization of PEA-PPEM systems, the transparent copolymers were obtained. The spectral transmittance of the poly(PEA-*co*-PPEM) networks is represented in **Figure 7.** The transmittances of copolymers are over 80 % in the visible light range.

### 2.5 Equilibrium water content (EWC)

The EWC of the copolymers ranged from 0.1% to 0.5%. These materials are hydrophobic in nature, so low EWC values were expected. The EWC values were measured on day 1 and day 5 after BSS immersion. No increase in EWC was seen after day 1.

### 2.6 Water contact angle

Water contact angles of the prepared copolymer networks presented in **Table 5,** ranges from 76.86 to 85.01°. The water drop profiles on the three representative samples with S2-EG-10, S2-EG-20 and S2-EG-30 were studied. It should also be noted that the surface wettability of the networks could be adjusted by adding the monomer PPEM with methyl groups, the more PPEM, higher hydrophobic surface can be obtained.

### 2.7 Unfolding rate analysis

Polymer discs of 10 mm diameter and 0.5 mm thickness were folded in half and amount of time for the polymer discs to return to their original shape were recorded. Each measurement was done three times and the average was taken. Unfolding times represented in **Table 5.** For the S2-EG-10 copolymer network, the recovery time is 3 sec. As the concentration of PPEM increases to 30 mol%, the recovery time is prolonged to 16 sec. When the concentration of PPEM reaches to 40 mol%, the samples could not recover their shapes to the original ones at 37 °C. ,

### 2.8 Mechanical tensile testing

**Figure 8** shows the stress plotted against its respective % strain determined from the mechanical tensile tests. Polymers having PPEM concentration below 25 mol% exhibiting the '*J*' shaped stress-strain curve. The curve shows that initially, small increases in stress give large extensions, however, at larger extensions the material becomes stiffer, and more difficult to extend. Copolymers S2-EG-30 and S2-HD-30 gives *'S'* shaped stress-strain curves, which are particularly susceptible to elastic instabilities. The tensile behavior was measured at 37 °C, which is beyond the $T_g$ of the networks. **Table 6** represents values of Young's Modulus at 100% strain, strength at failure strain and % elongation of synthesized polymer networks. From data it is observed that % elongation for poly(PEA-*co*-PPEM) networks is less than in comparison with poly(PEA-*co*-PPEA) networks, while stength at failure strain (stiffness) is more.

**Table 6: Mechanical tensile testing parameters for poly(PEA-*co*-PPEM) networks**

| Code | Young's Modulus at 100 % strain (MPa) | Strength at failure strain (MPa) | % Elongation |
|---|---|---|---|
| S2-EG-10 | 0.006 | 1.236 | 142 |
| S2-EG-15 | 0.006 | 1.164 | 135 |
| S2-EG-20 | 0.008 | 2.690 | 161 |
| S2-EG-25 | 0.009 | 4.363 | 180 |
| S2-EG-30 | 0.060 | 6.856 | 100 |
| S2-HD-20 | 0.015 | 2.545 | 119 |
| S2-HD-30 | 0.040 | 5.899 | 113 |

### 2.9 Cytotoxicity

The microscopy images were taken in bright field at 10X magnification obtained after incubation with polymer networks, in comparison with the negative control and positive controls. Live L929 mouse fibroblast adherent cells can propagate to a confluent monolayer with the increase in the culture time, as could be seen from those of negative control. The death of the cell was observed using a positive control, the open area between cells indicated that cell lysis has

occurred. In contrast, the fibroblast L929 cells incubated with polymer networks maintained their morphology typical of L929. No cell debris and no detachment from dish bottom was observed. Results regarding cell viability (MTT assay) of cultures are shown in Figure 9. L929 cells presented a high proliferation rate throughout the culture time. At early incubation times, that is, day 1, values of MTT reduction were similar in seeded networks and negative controls, suggesting an identical number of attached cells, whereas the cell viability of the positive control went down to ~19 % in one day and almost to 0 % in two days, At day 7, the cell viability was stable (almost 100 %) for the negative control, whereas for the networks, it was a bit lower than that of the negative control and much higher than that of the positive control.

These results suggested that copolymers developed in the study were cytocompatible. This slight toxicity, which turned out to be favorable, could prevent or reduce lens epithelial cell proliferation without any damage to other ocular tissues, because the IOL materials was confined within the capsule.

### 2.10 Thermogravimetric analysis (TGA)

TGA curves for representative polymers represented in **Figure10.** Thermograms indicate that polymer networks are thermally stable and decomposition of polymers starts at 310 °C. Single step degradation observed in all copolymers.

### Example 3: Synthesis of copolymer networks of 2-phenylethyl acrylate (PEA) and 2-phenyl-(2-phenylthio)ethyl acrylate (PTEA)

Poly(PEA-*co*-PTEA) networks were prepared by radical copolymerisation of 2-phenylethyl acrylate (PEA) and 2-phenyl-(2-phenylthio)ethyl acrylate (PTEA) in the presence of 2 mol % ethylene dimethacrylate (EGDMA) or hexanediol dimethacrylate (HDDMA) as a cross-linker, and 0.2 mol% Luperox-256 as an initiator. The networks were prepared with varying molar ratio of PEA and PTEA. Monomers used and co-polymerization feed compositions are detailed in **Table 7.** Around 5 g monomer mixtures were weighed in centrifuge tubes and the appropriate amount of each component was added. Mixtures were vortexed for 60 seconds to allow the homogenization. Nitrogen gas was bubbled through the mixtures to remove most of the oxygen present. Mixtures were then syringed into a glass molds. The glass molds were placed in an oven at 60 °C for 20 h followed by 90 °C for 10 h to ensure complete polymerization. After polymerization, the cross-linked poly(PEA-*co*-PTEA) networks were extracted using soxhlet extraction with 2-propanol to remove unreacted monomers and oligomers until equilibrium was reached.

**Table 7: Monomer compositions for PEA-PTEA bulk polymerisation system**

| Sr. No. | Code | PEA (mol %) | PTEA (mol %) | Cross-linker mol% | % Wt. loss after solvent extraction |
|---|---|---|---|---|---|
| 1 | S3-EG-20 | 78.4 | 19.6 | 2 mol % EGDMA | 1.4 |
| 2 | S3-EG-30 | 68.6 | 29.4 | 2 mol % EGDMA | 1.1 |
| 3 | S3-EG-40 | 58.8 | 39.2 | 2 mol % EGDMA | 1.0 |
| 4 | S3-EG-50 | 49 | 49 | 2 mol % EGDMA | 0.9 |
| 5 | S3-EG-60 | 39.2 | 58.8 | 2 mol % EGDMA | 0.9 |
| 6 | S3-HD-40 | 58.8 | 39.2 | 2 mol % HDDMA | 1.0 |
| 7 | S3-HD-50 | 49 | 49 | 2 mol % HDDMA | 1.1 |

### 3.1 IR Spectroscopy

IR spectra of representative copolymers in comparison with monomer spectra indicated complete polymerization as absorption peak of C=C double bond at 1636 $cm^{-1}$ can be clearly observed in the monomers but disappears in the copolymer.

### 3.2 Differential scanning calorimetry (DSC)

The Glass transition temperatures ($T_g$) of the polymers represented in **Figure 11.** All of the cured acrylate resins show $T_g$'s in the range of 6.10 to 18.26 °C measured by DSC. The increased concentration of PTEA containing two aromatic rings substantially reduces the mobility of polymer chains within the networks, as can be seen from the increase in the $T_g$ represented in **Table 8.** Poly(PEA-*co*-PTEA) networks exhibiting lower $T_g$ values than corresponding oxygen containing poly(PEA-*co*-PPEA) networks.

**Table 8: Refractive index, glass transition temperature, water contact angle and unfolding time of poly(PEA-*co*-PTEA) networks**

| Sr. No. | Code | R.I. (at 589 nm) | $T_g$ (°C) | Contact Angle | Unfolding Time (sec) |
|---|---|---|---|---|---|
| 1 | S3-EG-20 | 1.573 | 6.10 | 84.38 | 5 |
| 2 | S3-EG-30 | 1.581 | 10.39 | 85.76 | 9 |
| 3 | S3-EG-40 | 1.588 | 13.77 | 83.23 | 8 |
| 4 | S3-EG-50 | 1.595 | 15.32 | 83.86 | 11 |
| 5 | S3-EG-60 | 1.601 | 18.26 | 84.51 | 15 |
| 6 | S3-HD-40 | 1.585 | 10.74 | 83.73 | 10 |
| 7 | S3-HD-50 | 1.590 | 11.98 | 83.82 | 8 |

### 3.3 Refractometry (R.I.)

Values of refractive indices for all sulphur containing polymer networks listed in **Table 8.** All resulting polymers exhibit significantly high-refractive indices compared with corresponding oxygen containing bulk polymer networks. RI increases from 1.573 to 1.601 with the increase in the concentration of PTEA in polymer.

### 3.4 Transmittance

Copolymers are clear and colorless even at higher mol % of PTEA (60 mol %). This transparency of the polymer networks is considered to be a result of uniform distribution of PTEA resulting homogeneous medium, having an increased refractive index and PTEA does not scatter the visible light. As shown in **Figure 12** all copolymers has a reasonable transmission (>85% ranging from 400 nm to 800 nm), which is comparable to the human lens and filters out most of the UV light.

### 3.5 Equilibrium water content (EWC)

The EWC of the copolymers ranged from 0.05% to 0.2%. These materials are hydrophobic in nature, so low EWC values were expected. The EWC values were measured on day 1 and day 5 after immersion into Balanced Salt Solution. No increase in EWC was seen after day 1.

### 3.6 Water contact angle

Contact angle measurements for all the copolymers can be found in **Table 8.** Contact angle values for polymer networks slightly differed from each other and ranged from 83.23 to 85.76°.

### 3.7 Unfolding rate analysis

Polymer discs of 10 mm diameter and 0.5 mm thickness were folded in half and amount of time for the polymer discs to return to their original shape were recorded. Each measurement was done three times and the average was taken. Unfolding times represented in **Table 8.** All the prepared copolymers could recover their original shape as time increased and the recovery ratios were 100% at the testing temperature. With the increasing concentrations of PTEA, the recovery time increased.

### 3.8 Mechanical tensile testing

**Figure 13** displays representative stress-strain curves of the seven copolymer networks. **Table 9** represents values of Young's Modulus at 100% strain, strength at failure strain and % elongation of synthesized polymer networks. Results indicate that as concentration of PTEA increases, the strength of material increases. All copolymer networks exhibit the '*J*-shaped' stress-strain curve, which is characteristic property of elastic rubbery polymers. The curve shows that initially, small increases in stress give large extensions, however, at larger extensions the material becomes stiffer, and more difficult to extend.

**Table 9: Mechanical tensile testing parameters for poly(PEA-*co*-PTEA) networks**

| Code | Young's Modulus at 100 % strain (MPa) | Strength at failure strain (MPa) | % Elongation |
|---|---|---|---|
| S3-EG-20 | 0.004 | 2.867 | 247 |
| S3-EG-30 | 0.004 | 3.421 | 269 |
| S3-EG-40 | 0.004 | 4.908 | 324 |
| S3-EG-50 | 0.004 | 6.863 | 348 |
| S3-EG-60 | 0.005 | 9.247 | 357 |
| S3-HD-40 | 0.007 | 2.320 | 154 |
| S3-HD-50 | 0.008 | 5.078 | 184 |

### 3.9 Cytotoxicity

Cytotoxicity of the prepared polymer networks to L929 mouse fibroblasts was tested. L929 mouse fibroblasts were cultured for 7 days in direct contact to the networks. The effects of the networks on cell morphology were investigated by Carl Zeiss Axio scope microscope. The the microscopy images were taken in bright field at 10X magnification obtained after incubation with polymer networks, in comparison with the negative control and positive controls. Live L929 mouse fibroblast adherent cells can propagate to a confluent monolayer with increase in the culture time, as could be seen from those of negative control. The death of the cell is observed using a positive control, the open area between cells indicates that cell lysis has occurred. In contrast, the fibroblast L929 cells incubated with polymer networks maintain their morphology typical of L929. No cell debris and no detachment from dish bottom is observed. Results regarding cell viability (MTT assay) of cultures are shown in **Figure 14.** L929 cells presented a high proliferation rate throughout the culture time. At early incubation times, that is, day 1, values of MTT reduction were similar in seeded networks and negative controls, suggesting an identical number of attached cells, whereas the cell viability of the positive control went down to ~19 % in one day and almost to 0 % in two days. On day 7, the cell viability was stable (almost 100 %) for the negative control, whereas for the networks, it was a bit lower than that of the negative control and much higher than that of the positive control. These results suggested a lack of Cytotoxicity of polymer networks developed in this study, which was critical for their biomedical application in the reduction of inflammatory response and PCO.

### 3.10 Thermogravimetric analysis (TGA)

TGA curves for representative polymers represented in **Figure 15.** Thermograms clearly indicate that polymers undergo two-stage decomposition. The initial decomposition temperature of polymers is ~290 °C, while second decomposition temperature is ~ 340 °C.

### Example 4: Synthesis of copolymer networks of 2-phenylethyl acrylate (PEA) and 2-phenyl-(2-phenylthio)ethyl methacrylate (PTEM)

Poly(PEA-*co*-PTEM) networks were prepared by radical copolymerisation of 2-phenylethyl acrylate (PEA) and 2-phenyl-(2-phenylthio)ethyl methacrylate (PTEM) in the presence of 2 mol % ethylene dimethacrylate (EGDMA) or hexanediol dimethacrylate, (HDDMA) as a cross-linker and 0.2 mol% Luperox-256 as an initiator. The networks were prepared with varying molar ratio of PEA and PTEM. Monomers used and co-polymerization feed compositions detailed in **Table 10.** Around 5 g monomer mixtures were weighed in centrifuge tubes and the appropriate amount of each component was added. Mixtures were vortexed for 60 seconds to allow the homogenization. Nitrogen gas was bubbled through mixtures to remove most of the oxygen present. Mixtures were then syringed into a glass molds. The glass molds were placed in an oven at 60 °C for 20 h followed by 90 °C for 10 h to ensure complete polymerization. After polymerization, the cross-linked poly(PEA-*co*-PTEM) networks were extracted using soxhlet extraction with 2-propanol to remove unreacted monomers and oligomers until equilibrium was reached.

**Table 10: Monomer compositions for PEA-PTEM bulk polymerisation system**

| Sr. No. | Code | PEA (mol %) | PTEM (mol %) | Cross-linker | % Wt. loss after solvent extraction |
|---|---|---|---|---|---|
| 1 | S4-EG-10 | 88.2 | 9.8 | 2 mol % EGDMA | 0.5 |

(continued)

| Sr. No. | Code | PEA (mol %) | PTEM (mol %) | Cross-linker | % Wt. loss after solvent extraction |
|---|---|---|---|---|---|
| 2 | S4-EG-15 | 83.3 | 14.7 | 2 mol % EGDMA | 1.3 |
| 3 | S4-EG-20 | 78.4 | 19.6 | 2 mol % EGDMA | 1.1 |
| 4 | S4-EG-25 | 73.5 | 24.5 | 2 mol % EGDMA | 1.1 |
| 5 | S4-EG-30 | 68.6 | 29.4 | 2 mol % EGDMA | 0.9 |
| 6 | S4-HD-20 | 78.4 | 19.6 | 2 mol % HDDMA | 1.4 |
| 7 | S4-HD-30 | 68.6 | 29.4 | 2 mol % HDDMA | 0.8 |

**4.1 IR spectroscopy**

The comparative FTIR spectra of two representative copolymers and corresponding monomers shows that the absorption peak of C=C double bond at 1637 $cm^{-1}$ can be observed clearly in the monomers, but disappears in the copolymers, which indicates that the C=C double bonds were almost exhausted during the polymerization.

**4.2 Differential scanning calorimetry (DSC)**

The glass transition curves of the polymer networks derived from DSC represented in **Figure 16.** $T_g$ for all five networks ranges between 2.84 and 21.67 °C. The glass transition temperatures of polymers were allowed to be optimized for intraocular lens application by adjusting the amounts of starting monomers without introducing any new monomers or additives into the system. The increased concentration of methacrylate substantially reduces the mobility of polymer chains within the networks, as can be seen from the increase in the $T_g$ shown in **Table 11.**

**Table 11: Refractive index, glass transition temperature, water contact angle and unfolding time of poly(PEA-*co*-PTEM) networks**

| Sr. No. | Codes | R.I. (at 589 nm) | $T_g$ (°C) | Contact Angle | Unfolding Time (sec) |
|---|---|---|---|---|---|
| 1 | S4-EG-10 | 1.559 | 2.84 | 81.74 | 4 |
| 2 | S4-EG-15 | 1.563 | 4.45 | 83.21 | 4 |
| 3 | S4-EG-20 | 1.567 | 11.22 | 84.80 | 6 |
| 4 | S4-EG-25 | 1.573 | 15.00 | 85.35 | 10 |
| 5 | S4-EG-30 | 1.578 | 21.67 | 87.35 | 19 |
| 6 | S4-HD-20 | 1.567 | 9.89 | 83.86 | 7 |
| 7 | S4-HD-30 | 1.577 | 21.45 | 86.46 | 18 |

**4.3 Refractometry**

RI of current acrylate foldable IOLs is approximately 1.51. Both the high RI and flexibility of the copolymers could be very helpful to reduce the implanted incision when they are used as IOLs. **Table 11** shows the values of RI of copolymers with different concentrations and compositions of the monomers. With increasing the concentrations of PTEM from 10 to 30 mol%, the RI increased from 1.559 to 1.578. The high RI of the copolymers resulted from sulphur atom and aromatic rings of PTEM.

**4.4 Transmittance**

After the polymerization of PEA-PTEM systems, the transparent copolymers were obtained. **Figure 17** shows the UV-vis absorption spectra of the poly(PEA-*co*-PTEA) networks with a thickness of about 0.5 mm. All polymers show high transparency in the visible region (A= 400-800 nm) with transmittance over 80% at 400 nm,_indicating that the introduction of a thiophenol unit does not deteriorate the optical transparency of the films. The thio-ether unit may suppress packing between the polymer chains, which is required for high transparency.

### 4.5 Equilibrium water content (EWC)

The EWC of the copolymers ranged from 0.1%-0.4%). These materials are hydrophobic in nature, so low EWC values were expected. The EWC values were measured on day 1 and day 5 after immersion into Balanced Salt Solution. No increase in EWC was seen after day 1.

### 4.6 Water contact angle

The water drop profiles of representative copolymer networks are shown in **Table 11.** It should also be noted that the surface wettability of the networks could be adjusted by adding the monomer PTEM with methyl groups: the more the PTEM, the higher hydrophobic the surface could be obtained **(Table 11).**

### 4.7 Unfolding rate analysis

Unfolding times for synthesized bulk polymer networks are represented in **Table** 11. Unfolding time is varying from 4 to 19 sec. The S4-EG-10 composition tended to adhere to itself for a brief moment before unfolding.

### 4.8 Mechanical tensile testing

**Figure 18** shows the stress plotted against its respective % strain determined from the mechanical tensile test. Polymers having PTEM concentration below 25 mol% exhibiting the '*J*' shaped stress-strain curve. The curve shows that initially, small increases in stress give large extensions, however, at larger extensions the material becomes stiffer, and more difficult to extend. Copolymers having PTEM content more than 25 mol% shows more stiffness and less elongation. The tensile behavior was measured at 37 °C, which is beyond the $T_g$ of the networks. **Table 12** represents values of Young's Modulus at 100% strain, strength at failure strain and % elongation of synthesized polymer networks.

**Table 12: Mechanical tensile testing parameters for poly(PEA-*co*-PTEM) networks**

| Code | Young's Modulus at 100 % strain (MPa) | Strength at failure strain (MPa) | % Elongation |
|---|---|---|---|
| S4-EG-10 | 0.007 | 2.390 | 176 |
| S4-EG-15 | 0.011 | 5.078 | 176 |
| S4-EG-20 | 0.011 | 6.795 | 225 |
| S4-EG-25 | 0.028 | 7.507 | 163 |
| S4-EG-30 | 0.051 | 12.696 | 135 |
| S4-HD-20 | 0.024 | 8.886 | 155 |
| S4-HD-30 | 0.045 | 11.193 | 136 |

### 4.9 Cytotoxicity

Cytotoxicity tests were performed with L929 mouse fibroblasts on the prepared networks. All the networks exhibit a favorable biocompatibility. The microscopy images were taken in bright field at 10X magnification obtained after incubation with polymer networks, in comparison with the negative and positive controls. Live L929 mouse fibroblast adherent cells can propagate to a confluent monolayer with the increase in the culture time, as could be seen from those of negative control. The death of the cell was observed using a positive control, the open area between cells indicated cell lysis had occurred. In contrast, the fibroblast L929 cells incubated with polymer networks maintained their morphology typical of L929. No cell debris and no detachment from dish bottom was observed. Results regarding cell viability (MTT assay) of cultures are shown in **Figure19.** L929 cells presented a high proliferation rate throughout the culture time. At early incubation times, that is, day 1, values of MTT reduction were similar in seeded networks and negative controls, suggesting an identical number of attached cells, whereas the cell viability of the positive control went down to ~ 19 % in one day and almost to 0 % in two days. At day 7, the cell viability was stable (almost 100 %) for the negative control, whereas for the networks, it was a bit lower than that of the negative control and much higher than that of the positive control. The slight drop of cell viability in the prepared networks may be due to the inherent cytotoxicity of the (meth)acrylate polymers. These results suggest favorable cytocompatibility of polymer networks prepared in the study.

### 4.10 Thermogravimetric analysis (TGA)

The thermal stability of selected polymer networks was probed using thermogravimetric analysis **(Figure 20).** Polymers showed single step degradation with high thermal stability. All polymer networks are thermally stable up to 290 °C.

## ADVANTAGES OF THE INVENTION

A new family of high refractive index, transparent and cyatocompatible rubbery biomaterials, has been developed to provide this next stage of intraocular lens evolution. The use of high refractive index materials would result in thinner IOLs, which in turn would result in a smaller incision made during the surgery, and hence shorter surgery duration and recovery period.

These novel IOL materials are formed from crosslinked poly(meth)acrylate and do not contain any groups that can cause adverse reactions in the surrounding tissue or detrimentally affect the clarity of the optic by crazing.

These new polymers are true rubbery elastomers and return to their original shape after deformation. They have a high index of refraction (1.55-1.60), a low glass transition temperature (<20 °C) a highly transparent in visible region (> 85%) a low modulus of elasticity (<2 MPa) and high elongation (> 150%), which renders them softer and flexible.

## Claims

1. A copolymer comprising 5 to 95 mol% monomer of formula I;

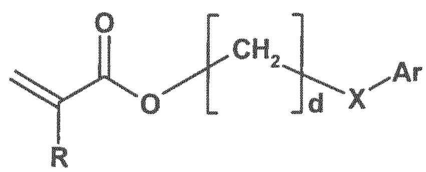

Formula I

wherein;

R is H or alkyl; 'd' is 1-10
X may be present or absent; with the proviso when X is present, X represents O, S or $NR^2$ where $R^2$ is H, (un)substituted or substituted alkyl, (un)substituted or substituted aryl, $CH_2C_6H_5$;
Ar represents an aromatic ring which is (un) substituted or substituted with H, $CH_3$, $CF_3$, $C_2H_5$, alkyl, $OCH_3$, $C_6H_{11}$, Cl, Br, $C_6H_5$, or $CH_2C_6H_5$;

5 to 95 mol% monomer of formula II;

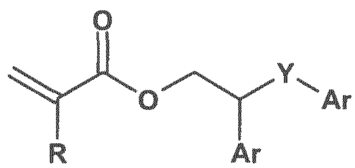

Formula II

wherein;

R is H or alkyl;
Y represents O or S;
Ar represent an aromatic ring which is (un) substituted or substituted with H, $CH_3$, $CF_3$, $C_2H_5$, alkyl, $OCH_3$, $C_6H_{11}$, Cl, Br, $C_6H_5$, or $CH_2C_6H_5$; and

and 0.1 to 20 mol% a co-polymerizable cross linker selected from the group consisting of terminally ethylenically unsaturated compounds such as hexanediol dimethacrylate (HDDMA), Ethylene glycol dimethacrylate (EGDMA),

allyl methacrylate, diallyl methacrylate; pentaerythritol tetra(meth)acrylate, 1,3-propanediol dimethacrylate, 1,4-butanediol dimethacrylate; and their corresponding acrylates.

2. The copolymer according to claim 1 comprising;

i. co-polymer of 2-phenylethyl acrylate (PEA), 2-phenoxy-2-phenylethyl acrylate (PPEA) and ethylene glycol dimethacrylate;
ii. co-polymer of 2-phenylethyl acrylate (PEA), 2-phenoxy-2-phenylethyl acrylate (PPEA) and hexanediol dimethacylate;
iii. co-polymer of 2-phenylethyl acrylate (PEA), 2-phenoxy-2-phenylethyl methacrylate (PPEM) and ethylene glycol dimethacrylate;
iv. co-polymer of 2-phenylethyl acrylate (PEA), 2-phenoxy-2-phenylethyl methacrylate (PPEM) and hexanediol dimethacylate;
v. co-polymer of 2-phenylethyl acrylate (PEA), 2-phenyl-(2-phenylthio)ethyl acrylate (PTEA) and ethylene glycol dimethacrylate;
vi. co-polymer of 2-phenylethyl acrylate (PEA), 2-phenyl-(2-phenylthio)ethyl acrylate (PTEA) and hexanediol dimethacylate;
vii. co-polymer of 2-phenylethyl acrylate (PEA), 2-phenyl-(2-phenylthio)ethyl methacrylate (PTEM) and ethylene glycol dimethacrylate; and
viii co-polymer of 2-phenylethyl acrylate (PEA), 2-phenyl-(2-phenylthio)ethyl methacrylate (PTEM) and hexanediol dimethacylate.

3. The copolymer according to claim 1, wherein said copolymer is hydrophobic and exhibit refractive index in the range of 1.558 to 1.601 (as determined in the experimental part).

4. The copolymer according to claim 1, wherein tensile strength of the said copolymer is in the range of 1.58 to 12.69 MPa (of 230 to 1841 psi, and as determined in the experimental part).

5. The copolymer according to claim 1, wherein said copolymer is flexible and exhibit modulus of elasticity in the range of 0.03 to 0.07 MPa (of 4 to 10 psi and as determined in the experimental part).

6. A process for the preparation of hydrophobic copolymer according to claim 1 by free radical copolymerization wherein the said process comprises the steps of:

i. mixing monomer I and monomer II in the ratio ranging between 5:95 to 95:5 mol% with cross linker and 0.1-5.0 mol% radical initiator in centrifuge tube and vortexing to allow homogenization;
ii. passing nitrogen gas through the mixture followed by injecting the mixture into the glass moulds;
iii. heating the glass moulds at 60°C for 20 h followed by isothermal heating at 90 °C for 10 h until complete polymerization; and
iv. extracting the cross-linked polymer network using soxhlet extraction with 2-propanol to remove the unreacted monomers and oligomers until equilibrium.

7. The co-polymer according to claims 1 and 2, wherein the monomer of formula (I) is selected from 2-phenylethyl acrylate (PEA), 2-phenoxyethyl methacrylate; 2-phenoxyethyl acrylate, benzyl acrylate, 3-phenylpropyl acrylate, 4-phenylbutyl acrylate, 2-(phenylthio)ethyl actylate, 2-(phenylamino)ethyl acrylate.

8. The co-polymer according to claims 1 and 2, wherein the monomer of formula (II) is selected from 2-phenoxy-2-phenylethyl acrylate (PPEA) or 2-phenoxy-2-phenylethyl methacrylate (PPEM) or 2-phenyl-(2-phenylthio)ethyl acrylate (PTEA) or 2-phenyl-(2-phenylthio)ethyl methacrylate (PTEM).

9. The co-polymer according to claims 1 to 5, wherein the cross linker is in the range of 0.1 to 20 mol% w.r.t the mol % of the combination of monomers.

10. The co-polymer according to any of the preceding claims optionally comprising additives selected from UV absorbers, dyes, light stabilizers, coating materials, pharmaceutical agents, cell receptor functional groups, viscosity agents, diluents or combinations thereof wherein the UV absorber used is in the range of 0.1 to 2.0 wt % of total monomers.

## Patentansprüche

1. Copolymer umfassend 5 bis 95 Mol-% Monomer der Formel I:

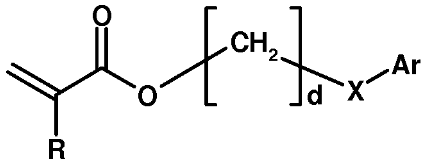

Formel I

wobei:

R H oder Alkyl ist; "d" 1 - 10 ist,
X vorhanden oder abwesend sein kann; mit der Maßgabe, dass, wenn X vorliegt, X O, S oder $NR^2$ darstellt, wobei $R^2$ H, (un)substituiertes oder substituiertes Alkyl, (un)substituiertes oder substituiertes Aryl, $CH_2C_6H_5$ ist;
Ar einen aromatischen Ring darstellt, der (un)substituiert oder mit H, $CH_3$, $CF_3$, $C_2H_5$, Alkyl, $OCH_3$, $C_6H_{11}$, Cl, Br, $C_6H_5$ oder $CH_2C_6H_5$ substituiert ist;

5 bis 95 Mol-% Monomer der Formel II:

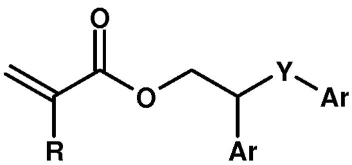

Formel II

wobei:

R H oder Alkyl ist;
Y O oder S darstellt;
Ar einen aromatischen Ring darstellt, der (un)substituiert oder mit H, $CH_3$, $CF_3$, $C_2H_5$, Alkyl, $OCH_3$, $C_6H_{11}$, Cl, Br, $C_6H_5$ oder $CH_2C_6H_5$ substituiert ist; und

und 0,1 bis 20 Mol-% eines copolymerisierbaren Vernetzungsmittels ausgewählt aus der Gruppe bestehend aus endständig ethylenisch ungesättigten Verbindungen wie Hexandioldimethacrylat (HDDMA), Ethylenglycoldimethacrylat (EGDMA), Allylmethacrylat, Diallylmethacrylat; Pentaerythrittetra(meth)acrylat, 1,3-Propandioldimethcrylat, 1,4-Butandioldimethacrylat; und ihren entsprechenden Acrylaten.

2. Copolymer nach Anspruch 1 umfassend:

i. Copolymer von 2-Phenylethylacrylat (PEA), 2-Phenoxy-2-phenylethylacrylat (PPEA) und Ethylenglycoldimethacrylat;
ii. Copolymer von 2-Phenylethylacrylat (PEA), 2-Phenoxy-2-phenylethylacrylat (PPEA) und Hexandioldimethacrylat;
iii. Copolymer von 2-Phenylethylacrylat (PEA), 2-Phenoxy-2-phenylethylmethacrylat (PPEM) und Ethylenglycoldimethacrylat;
iv. Copolymer von 2-Phenylethylacrylat (PEA), 2-Phenoxy-2-phenylethylmethacrylat (PPEM) und Hexandioldimethacrylat;
v. Copolymer von 2-Phenylethylacrylat (PEA), 2-Phenyl-(2-phenylthio)ethylacrylat (PTEA) und Ethylenglycoldimethacrylat;
vi. Copolymer von 2-Phenylethylacrylat (PEA), 2-Phenyl-(2-phenylthio)ethylacrylat (PTEA) und Hexandioldimethacrylat;

vii. Copolymer von 2-Phenylethylacrylat (PEA), 2-Phenyl-(2-phenylthio)ethylmethacrylat (PTEM) und Ethylenglycoldimethacrylat; und

viii Copolymer von 2-Phenylethylacrylat (PEA), 2-Phenyl-(2-phenylthio)ethylmethacrylat (PTEM) und Hexandioldimethacrylat.

**3.** Copolymer nach Anspruch 1, wobei das Copolymer hydrophob ist und einen Brechungsindex im Bereich von 1,558 bis 1,601 (wie im Versuchsteil bestimmt) aufweist.

**4.** Copolymer nach Anspruch 1, wobei die Zugfestigkeit des Copolymers im Bereich von 1,58 bis 12,69 MPa (230 bis 1841 psi und wie im Versuchsteil bestimmt) liegt.

**5.** Copolymer nach Anspruch 1, wobei das Copolymer flexibel ist und einen Elastizitätsmodul im Bereich von 0,03 bis 0,07 MPa (4 bis 10 psi und wie im Versuchsteil bestimmt) aufweist.

**6.** Verfahren für die Herstellung eines hydrophoben Copolymers nach Anspruch 1 durch Copolymerisation mit freien Radikalen, wobei das Verfahren die Schritte umfasst des:

i. Mischens von Monomer I und Monomer II im Verhältnis im Bereich von 5:95 bis 95:5 Mol-% mit Vernetzungsmittel und 0,1 - 5,0 Mol-% Radikalinitiator in einem Zentrifugenglas und Vortexens, um die Homogenisierung zu gestatten;

ii. Hindurchführens von Stickstoffgas durch die Mischung, gefolgt von Injizieren der Mischung in die Glasformen;

ii. Erhitzens der Glasformen bei 60°C 20 h lang, gefolgt von isothermischem Erhitzen bei 90 °C 10 h lang, bis zur vollständigen Polymerisation; und

iv. Extrahierens des vernetzten Polymernetzwerks unter Anwendung von Soxhlet-Extraktion mit 2-Propanol, um die unreagierten Monomere und Oligomere bis zum Äquilibrium zu entfernen.

**7.** Copolymer nach Anspruch 1 und 2, wobei das Monomer der Formel (I) unter 2-Phenylethylacrylat (PEA), 2-Phenoxyethylmethacrylat; 2-Phenoxyethylacrylat, Benzylacrylat, 3-Phenylpropylacrylat, 4-Phenylbutylacrylat, 2-(Phenylthio)ethylacrylat, 2-(Phenylamino)ethylacrylat ausgewählt wird.

**8.** Copolymer nach den Ansprüchen 1 und 2, wobei das Monomer der Formel (II) unter 2-Phenoxy-2-phenylethylacrylat (PPEA) oder 2-Phenoxy-2-phenylethylmethacrylat (PPEM) oder 2-Phenyl-(2-phenylthio)ethylacrylat (PTEA) oder 2-Phenyl-(2-phenylthio)ethylmethacrylat (PTEM) ausgewählt wird.

**9.** Copolymer nach den Ansprüchen 1 bis 5, wobei das Vernetzungsmittel im Bereich von 0,1 bis 20 Mol-% mit Bezug auf die Mol-% der Kombination von Monomeren liegt.

**10.** Copolymer nach einem der vorhergehenden Ansprüche, wahlweise Zusatzmittel umfassend ausgewählt unter UV-Absorptionsmitteln, Farbstoffen, Lichtstabilisatoren, Beschichtungsmaterialien, Pharmazeutika, funktionellen Zellrezeptorgruppen, Viskositätsmitteln, Verdünnungsmitteln oder Kombinationen davon, wobei das verwendete UV-Absorptionsmittel im Bereich von 0,1 bis 2,0 Gew.-% gesamter Monomere liegt.

**Revendications**

**1.** Copolymère comprenant de 5 à 95 % en mole du monomère de formule I :

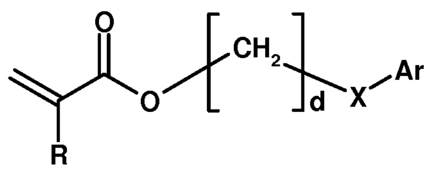

Formule 1

dans laquelle :

R est H ou alkyle; « d » a la valeur de 1 à 10 ;
X peut être présent ou absent; à condition que lorsque X est présent, X représente O, S ou $NR^2$ où $R^2$ est un atome H, un groupe alkyle (non)substitué ou substitué, un groupe aryle (non)substitué ou substitué, $CH_2C_6H_5$;
Ar représente un cycle aromatique qui est (non)substitué ou substitué par un atome H, un groupe $CH_3$, $CF_3$, $C_2H_5$, alkyle, $OCH_3$, $C_6H_{11}$, Cl, Br, $C_6H_5$, ou $CH_2C_6H_5$;

de 5 à 95 % en mole du monomère de formule II :

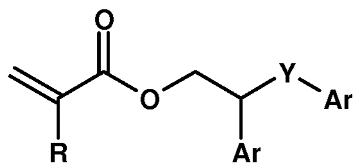

Formule II

dans laquelle :

R est H ou alkyle;
Y représente O ou S;
Ar représente un cycle aromatique qui est (non)substitué ou substitué par un atome H, un groupe $CH_3$, $CF_3$, $C_2H_5$, alkyle, $OCH_3$, $C_6H_n$, Cl, Br, $C_6H_5$, ou $CH_2C_6H_5$; et

et de 0,1 à 20 % en mole d'un agent de réticulation copolymérisable sélectionné dans le groupe constitué des composés éthyléniquement insaturés au plan terminal tels que le diméthacrylate d'hexanediol (HDDMA), le diméthacrylate d'éthylène glycol (EGDMA), le méthacrylate d'allyle, le méthacrylate de diallyle; le tétra(méth)acrylate de pentaérythritol, le diméthacrylate de 1,3-propanediol, le diméthacrylate de 1,4-butanediol; et leurs acrylates correspondants.

**2.** Copolymère selon la revendication 1 comprenant;

i. copolymère d'acrylate de 2-phényléthyle (PEA), d'acrylate de 2-phénoxy-2-phényléthyle (PPEA) et de diméthacrylate d'éthylène glycol;
ii. copolymère d'acrylate de 2-phényléthyle (PEA), d'acrylate de 2-phénoxy-2-phényléthyle (PPEA) et de diméthacrylate d'hexanediol;
iii. copolymère d'acrylate de 2-phényléthyle (PEA), de méthacrylate de 2-phénoxy-2-phényléthyle (PPEM) et de diméthacrylate d'éthylène glycol;
iv. copolymère d'acrylate de 2-phényléthyle (PEA), de méthacrylate de 2-phénoxy-2-phényléthyle (PPEM) et de diméthacrylate d'hexanediol;
v. copolymère d'acrylate de 2-phényléthyle (PEA), d'acrylate de 2-phényl-(2-phénylthio)éthyle (PTEA) et de diméthacrylate d'éthylène glycol;
vi. copolymère d'acrylate de 2-phényléthyle (PEA), d'acrylate de 2-phényl-(2-phénylthio)éthyle (PTEA) et de diméthacrylate d'hexanediol;
vii. copolymère d'acrylate de 2-phényléthyle (PEA), de méthacrylate de 2-phényl-(2-phénylthio)éthyle (PTEM) et de diméthacrylate d'éthylène glycol; et
viii. copolymère d'acrylate de 2-phényléthyle (PEA), de méthacrylate de 2-phényl-(2-phénylthio)éthyle (PTEM) et de diméthacrylate d'hexanediol.

**3.** Copolymère selon la revendication 1, dans lequel ledit copolymère est hydrophobe et fait preuve d'un indice de réfraction situé dans la plage de 1,558 à 1,601 (tel que déterminé dans la partie expérimentale).

**4.** Copolymère selon la revendication 1, dans lequel la résistance à la traction dudit copolymère se situe dans la plage de 1,58 à 12,69 MPa (de 230 à 1 841 lb/po$^2$, et telle que déterminée dans la partie expérimentale).

**5.** Copolymère selon la revendication 1, dans lequel ledit copolymère est souple et fait preuve d'un module d'élasticité situé dans la plage de 0,03 à 0,07 MPa (de 4 à 10 lb/po$^2$, et tel que déterminé dans la partie expérimentale).

**6.** Procédé de préparation d'un copolymère hydrophobe selon la revendication 1 par copolymérisation par radicaux libres, ledit procédé comprenant les étapes de:

i. mélange du monomère I et du monomère II sous le rapport s'étendant entre 5:95 à 95:5 % en mole avec l'agent de réticulation et de 0,1 à 5,0 % en mole d'initiateur de radicaux dans un tube de centrifugation et la mise sous vortex pour permettre l'homogénéisation;
ii. passage d'azote gazeux à travers le mélange suivi de l'injection du mélange dans les moules en verre;
iii. chauffage des moules en verre à 60°C durant 20 h suivi du chauffage isothermique à 90°C durant 10 h jusqu'à la polymérisation complète; et
iv. extraction du réseau de polymère réticulé en utilisant l'extraction de soxhlet avec du 2-propanol pour retirer les monomères et les oligomères n'ayant pas réagi jusqu'à l'équilibre.

**7.** Copolymère selon les revendications 1 et 2, dans lequel le monomère de formule (I) est sélectionné parmi l'acrylate de 2-phényléthyle (PEA), le méthacrylate de 2-phénoxyéthyle; l'acrylate de 2-phénoxyéthyle, l'acrylate de benzyle, l'acrylate de 3-phénylpropyle, l'acrylate de 4-phénylbutyle, l'acrylate de 2-(phénylthio)éthyle, l'acrylate de 2-(phénylamino)éthyle.

**8.** Copolymère selon les revendications 1 et 2, dans lequel le monomère de formule (II) est sélectionné parmi l'acrylate de 2-phénoxy-2-phényléthyle (PPEA) ou le méthacrylate de 2-phénoxy-2-phényléthyle (PPEM) ou l'acrylate de 2-phényl-(2-phénylthio)éthyle (PTEA) ou le méthacrylate de 2-phényl-(2-phénylthio)éthyle (PTEM).

**9.** Copolymère selon les revendications 1 à 5, dans lequel l'agent de réticulation se situe dans la plage de 0,1 à 20 % en mole en référence au % en mole de la combinaison des monomères.

**10.** Copolymère selon l'une quelconque des revendications précédentes comprenant éventuellement des additifs sélectionnés parmi les agents d'absorption du rayonnement UV, les colorants, les agents de stabilisation de la lumière, les matériaux d'enrobage, les agents pharmaceutiques, les groupes fonctionnels de récepteurs cellulaires, les agents de viscosité, les diluants ou leurs combinaisons, l'agent d'absorption du rayonnement UV utilisé se situant dans la plage de 0,1 à 2,0 % en pds des monomères totaux.

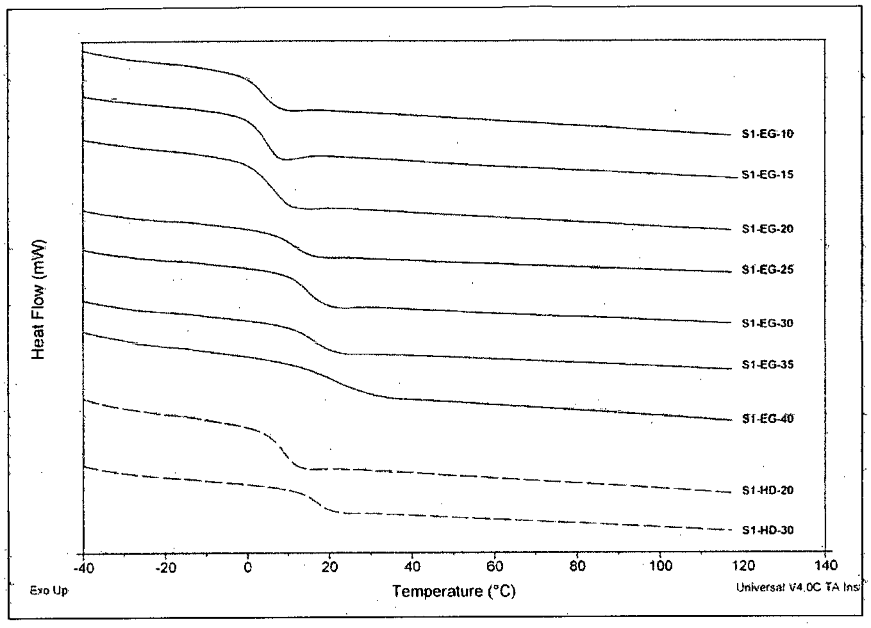
Heat Flow (mW)
S1-EG-10
S1-EG-15
S1-EG-20
S1-EG-25
S1-EG-30
S1-EG-35
S1-EG-40
S1-HD-20
S1-HD-30
-40 -20 0 20 40 60 80 100 120 140
Exo Up
Temperature (°C)
Universal V4.0C TA Ins

Fig 1

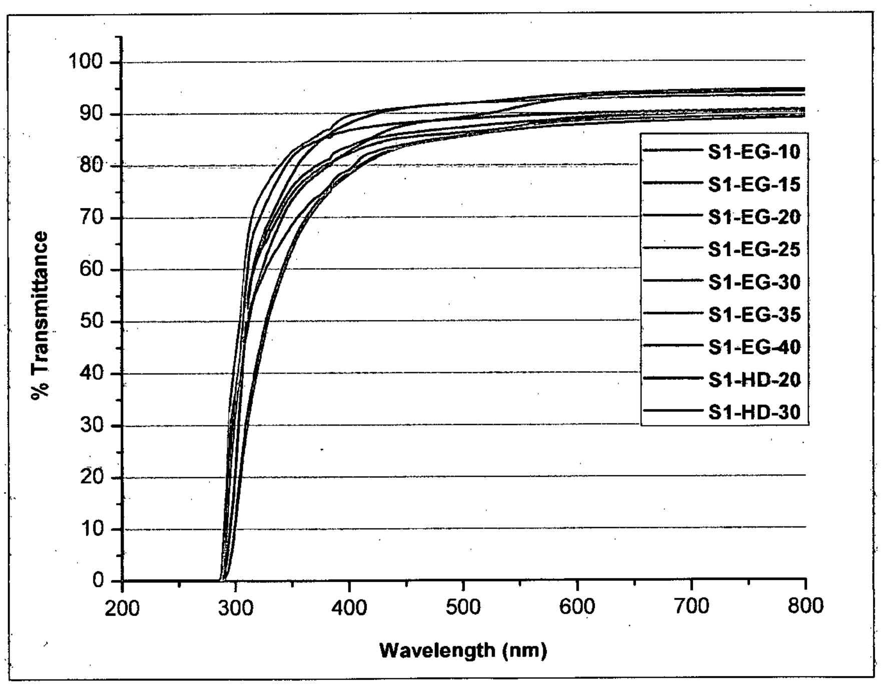
% Transmittance
100
90
80
70
60
50
40
30
20
10
0
200 300 400 500 600 700 800
Wavelength (nm)
S1-EG-10
S1-EG-15
S1-EG-20
S1-EG-25
S1-EG-30
S1-EG-35
S1-EG-40
S1-HD-20
S1-HD-30

Fig 2

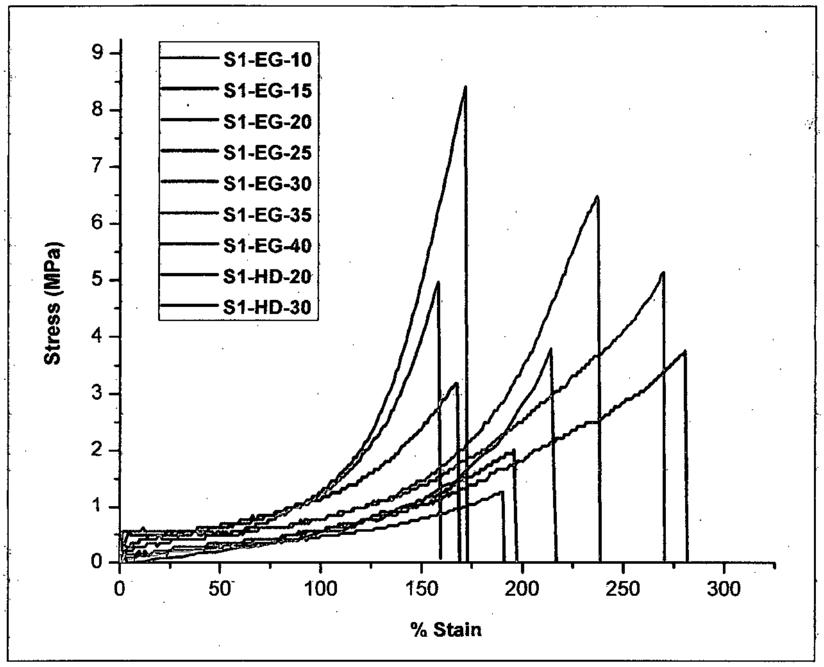
S1-EG-10
S1-EG-15
S1-EG-20
S1-EG-25
S1-EG-30
S1-EG-35
S1-EG-40
S1-HD-20
S1-HD-30
Stress (MPa)
% Stain
0
1
2
3
4
5
6
7
8
9
0
50
100
150
200
250
300

Fig 3

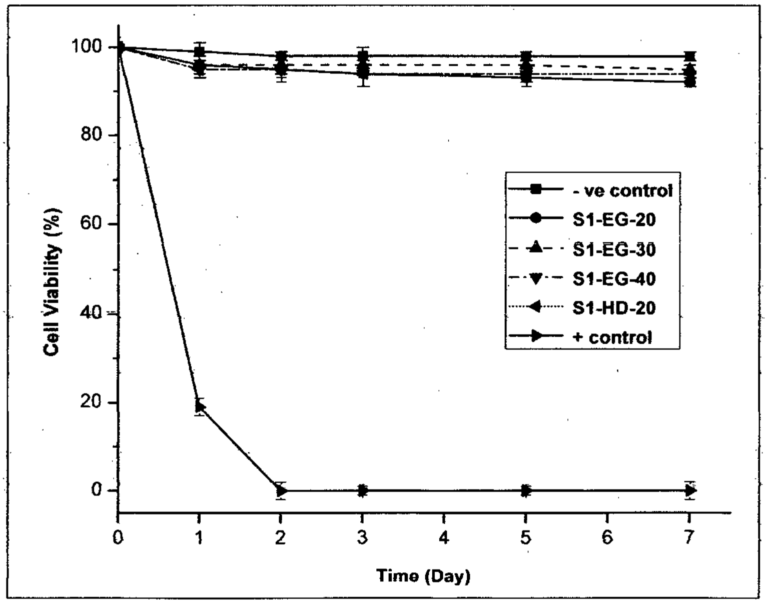
- ve control
S1-EG-20
S1-EG-30
S1-EG-40
S1-HD-20
+ control
Cell Viability (%)
Time (Day)
0
20
40
60
80
100
0
1
2
3
4
5
6
7

Fig 4

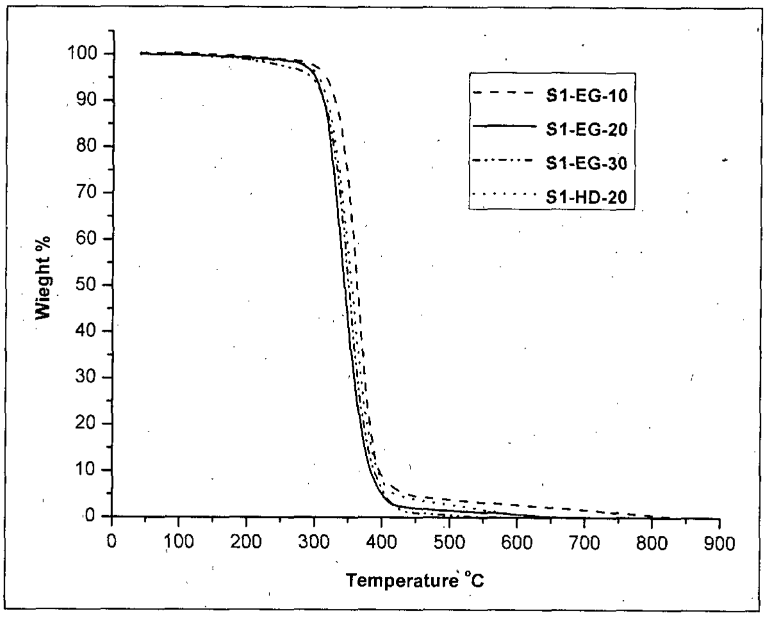
Wieght %
100
90
80
70
60
50
40
30
20
10
0
0
100
200
300
400
500
600
700
800
900
Temperature °C
S1-EG-10
S1-EG-20
S1-EG-30
S1-HD-20

**Fig 5**

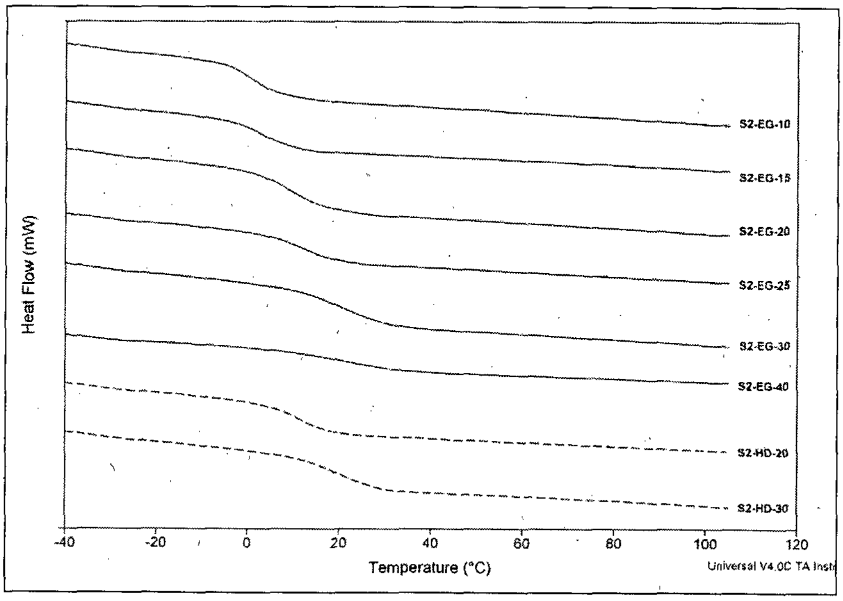
Heat Flow (mW)
S2-EG-10
S2-EG-15
S2-EG-20
S2-EG-25
S2-EG-30
S2-EG-40
S2-HD-20
S2-HD-30
-40
-20
0
20
40
60
80
100
120
Temperature (°C)
Universal V4.0C TA Instr

**Fig 6**

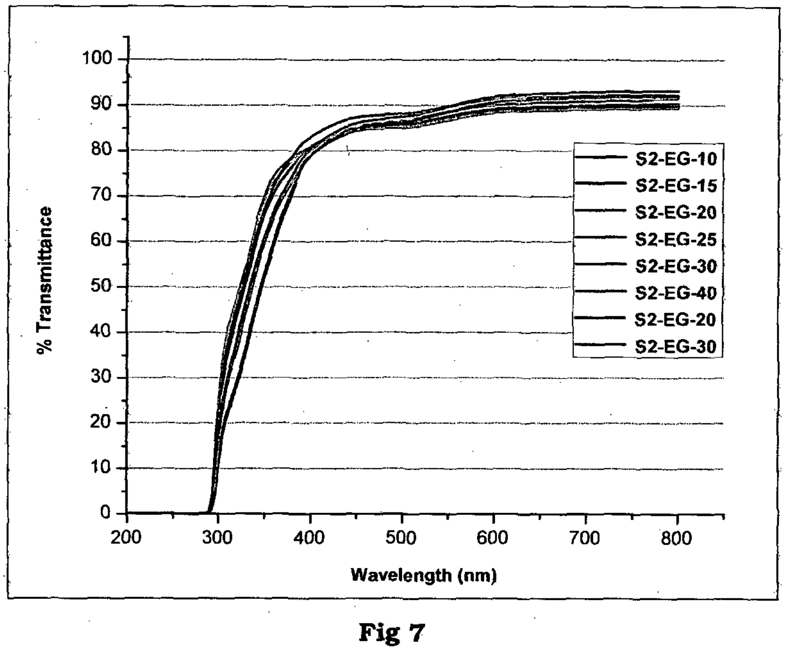
100
90
80
70
60
50
40
30
20
10
0
% Transmittance
200
300
400
500
600
700
800
Wavelength (nm)
S2-EG-10
S2-EG-15
S2-EG-20
S2-EG-25
S2-EG-30
S2-EG-40
S2-EG-20
S2-EG-30

Fig 7

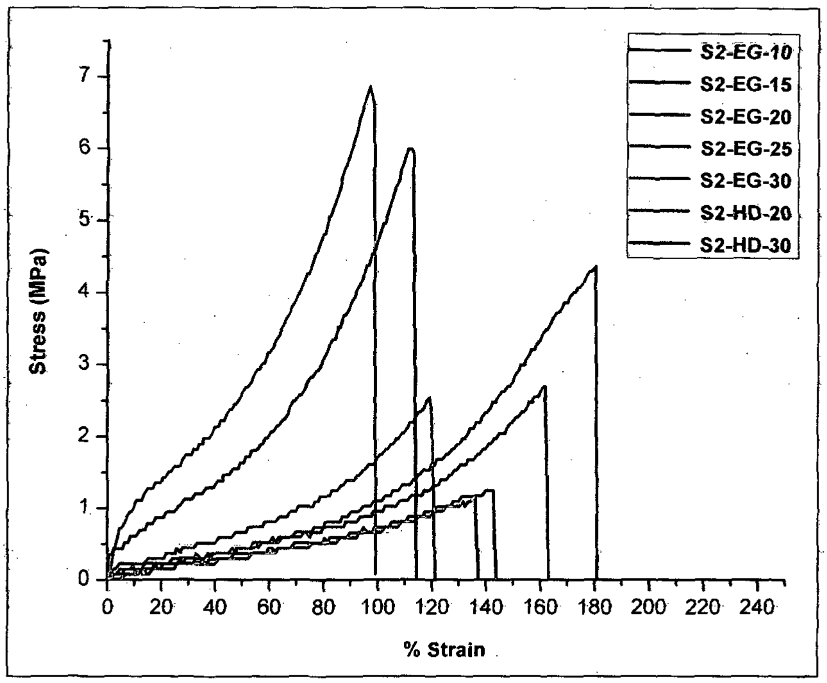
7
6
5
4
3
2
1
0
Stress (MPa)
0
20
40
60
80
100
120
140
160
180
200
220
240
% Strain
S2-EG-10
S2-EG-15
S2-EG-20
S2-EG-25
S2-EG-30
S2-HD-20
S2-HD-30

Fig 8

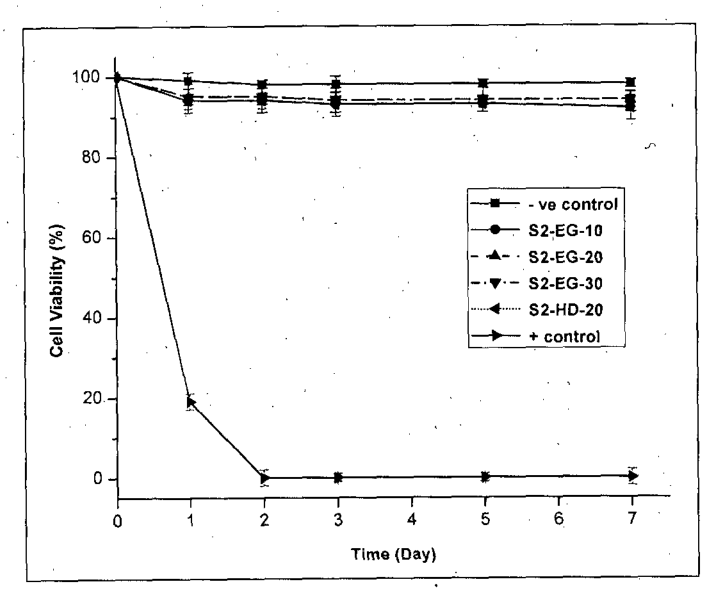
100
80
60
40
20
0
Cell Viability (%)
0
1
2
3
4
5
6
7
Time (Day)
- ve control
S2-EG-10
S2-EG-20
S2-EG-30
S2-HD-20
+ control

**Fig 9**

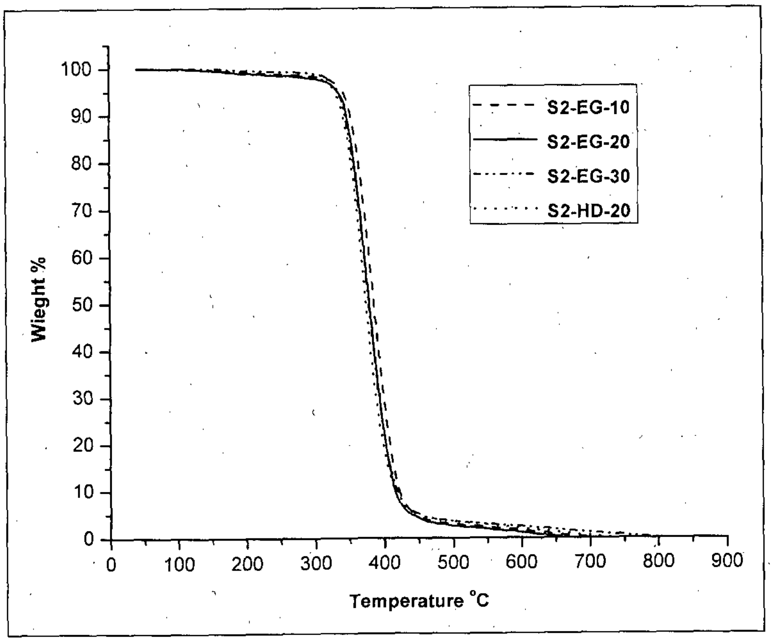
100
90
80
70
60
50
40
30
20
10
0
Wieght %
0
100
200
300
400
500
600
700
800
900
Temperature °C
S2-EG-10
S2-EG-20
S2-EG-30
S2-HD-20

**Fig 10**

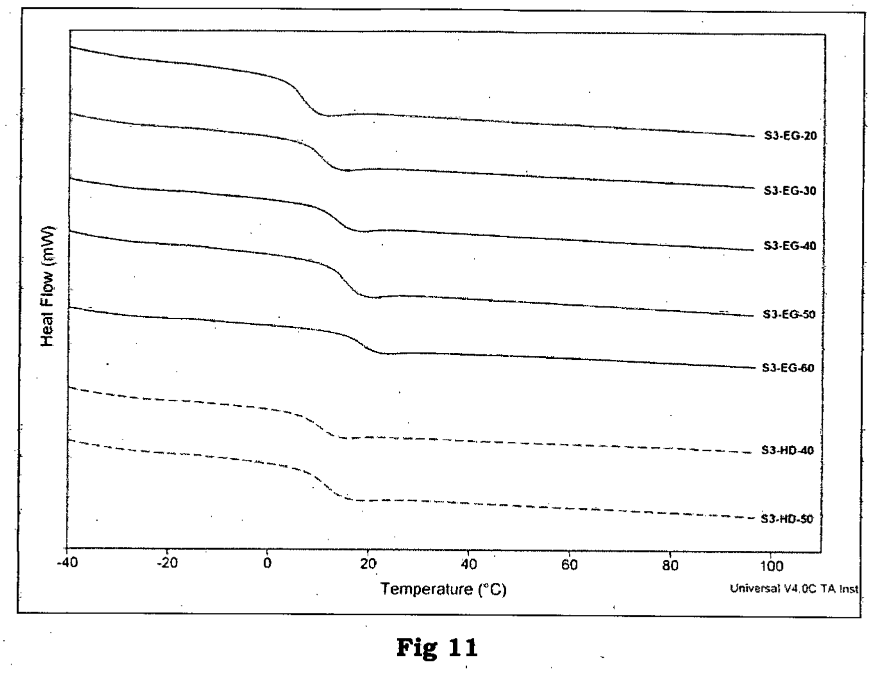
Heat Flow (mW)
S3-EG-20
S3-EG-30
S3-EG-40
S3-EG-50
S3-EG-60
S3-HD-40
S3-HD-50
-40
-20
0
20
40
60
80
100
Temperature (°C)
Universal V4.0C TA Inst

Fig 11

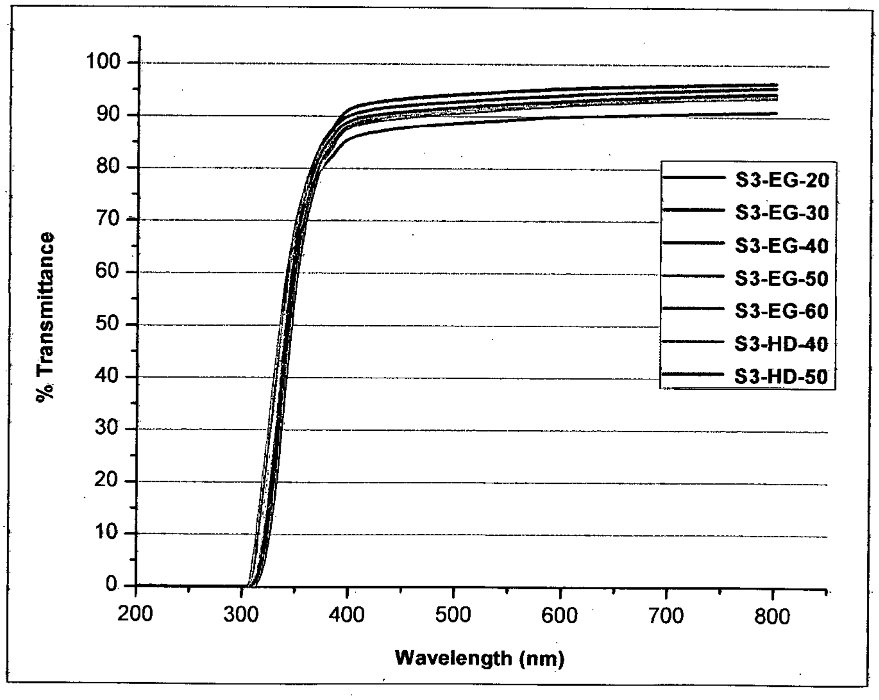
% Transmittance
100
90
80
70
60
50
40
30
20
10
0
200
300
400
500
600
700
800
Wavelength (nm)
S3-EG-20
S3-EG-30
S3-EG-40
S3-EG-50
S3-EG-60
S3-HD-40
S3-HD-50

Fig 12

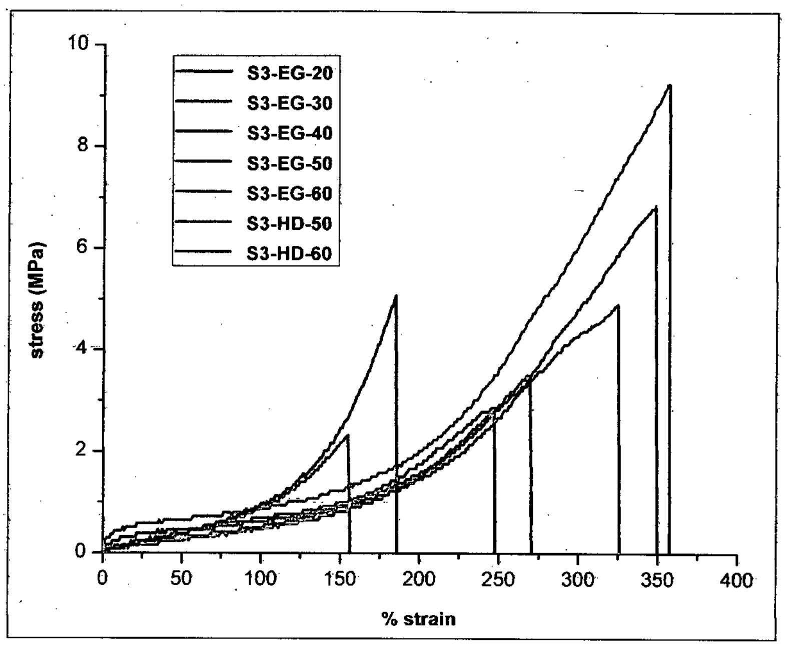
stress (MPa)
10
8
6
4
2
0
S3-EG-20
S3-EG-30
S3-EG-40
S3-EG-50
S3-EG-60
S3-HD-50
S3-HD-60
0
50
100
150
200
250
300
350
400
% strain

Fig 13

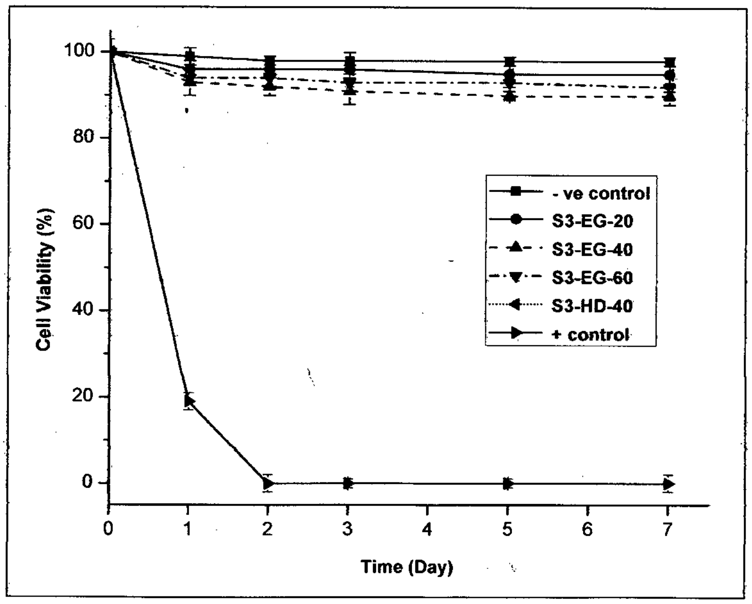
Cell Viability (%)
100
80
60
40
20
0
- ve control
S3-EG-20
S3-EG-40
S3-EG-60
S3-HD-40
+ control
0
1
2
3
4
5
6
7
Time (Day)

Fig 14

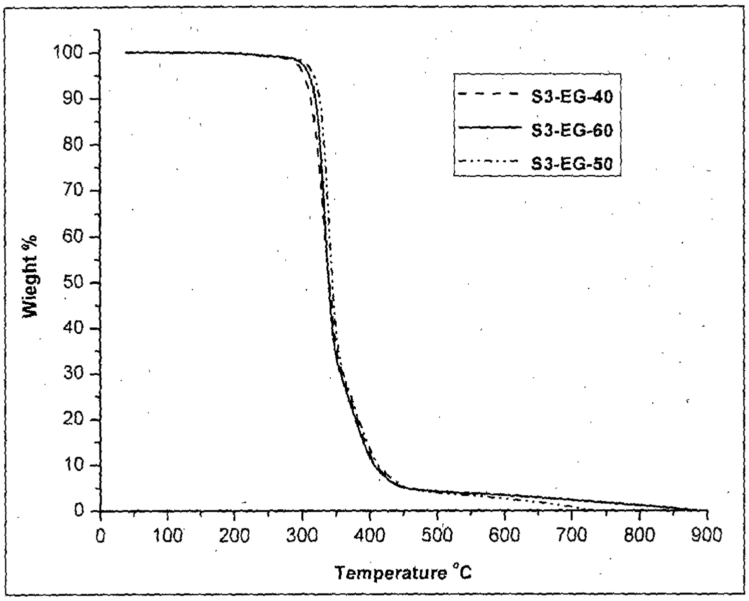
S3-EG-40
S3-EG-60
S3-EG-50
Wieght %
Temperature °C
100
90
80
70
60
50
40
30
20
10
0
0
100
200
300
400
500
600
700
800
900

Fig 15

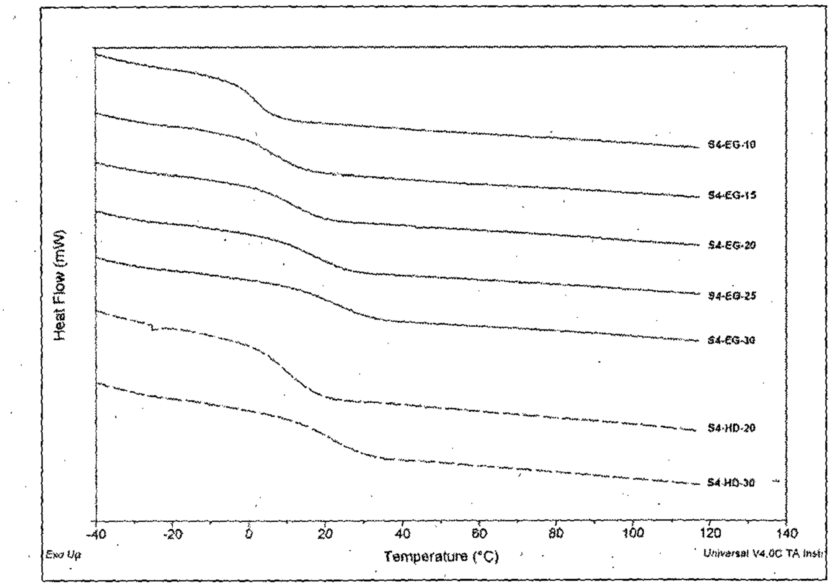
S4-EG-10
S4-EG-15
S4-EG-20
S4-EG-25
S4-EG-30
S4-HD-20
S4-HD-30
Heat Flow (mW)
Temperature (°C)
Exo Up
Universal V4.0C TA Instr
-40
-20
0
20
40
60
80
100
120
140

Fig 16

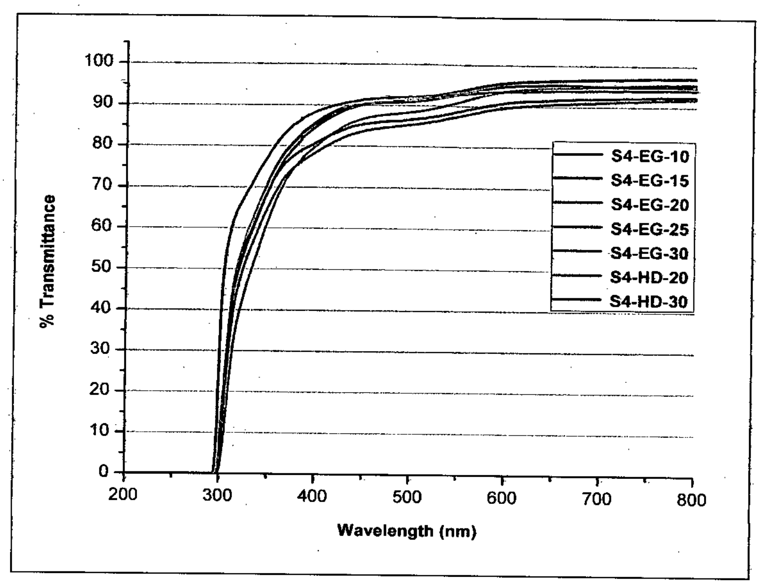
% Transmittance
100
90
80
70
60
50
40
30
20
10
0
200
300
400
500
600
700
800
Wavelength (nm)
S4-EG-10
S4-EG-15
S4-EG-20
S4-EG-25
S4-EG-30
S4-HD-20
S4-HD-30

**Fig 17**

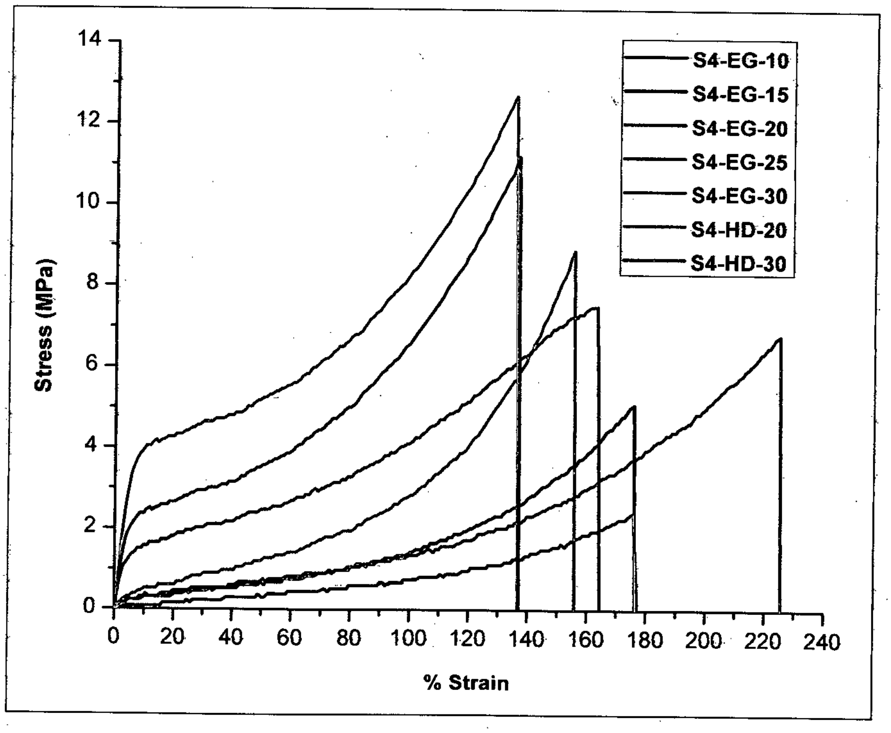
Stress (MPa)
14
12
10
8
6
4
2
0
0
20
40
60
80
100
120
140
160
180
200
220
240
% Strain
S4-EG-10
S4-EG-15
S4-EG-20
S4-EG-25
S4-EG-30
S4-HD-20
S4-HD-30

**Fig 18**

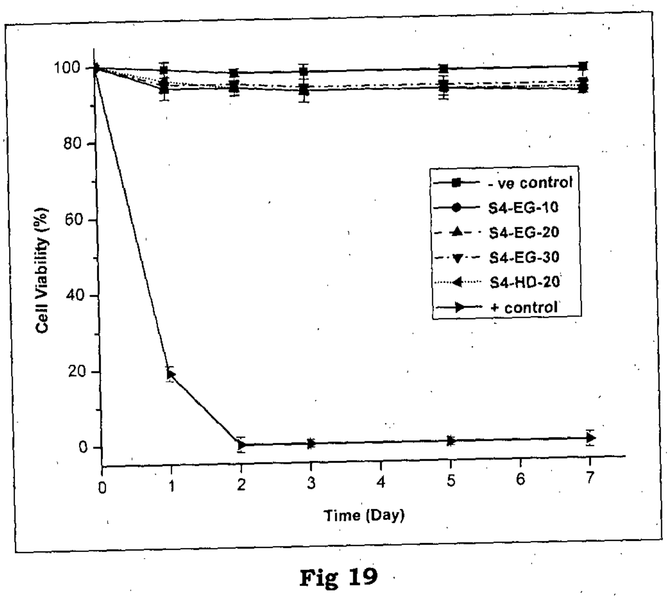
100
80
60
40
20
0
Cell Viability (%)
0
1
2
3
4
5
6
7
Time (Day)
- ve control
S4-EG-10
S4-EG-20
S4-EG-30
S4-HD-20
+ control

Fig 19

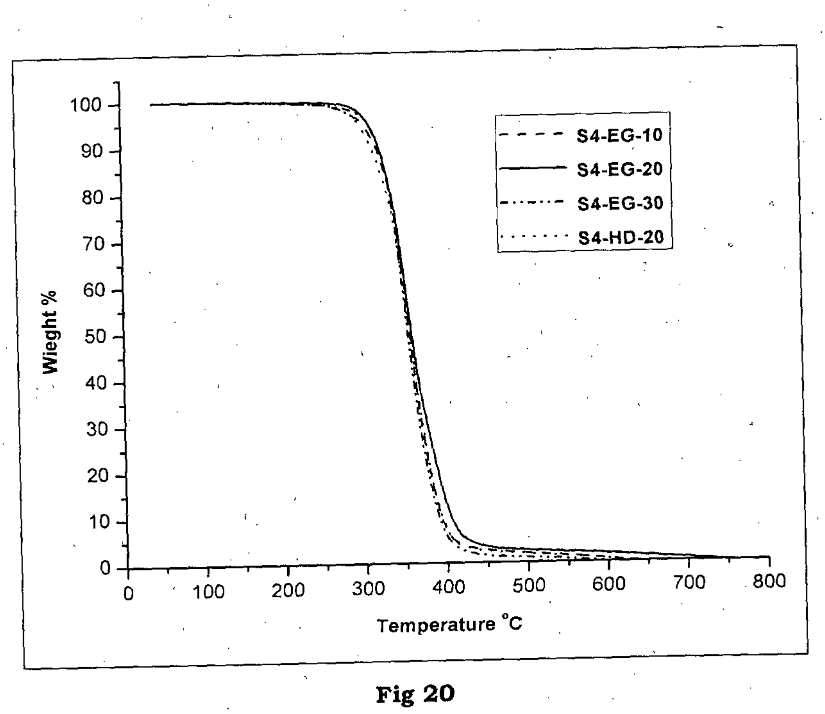
100
90
80
70
60
50
40
30
20
10
0
Wieght %
0
100
200
300
400
500
600
700
800
Temperature °C
S4-EG-10
S4-EG-20
S4-EG-30
S4-HD-20

Fig 20

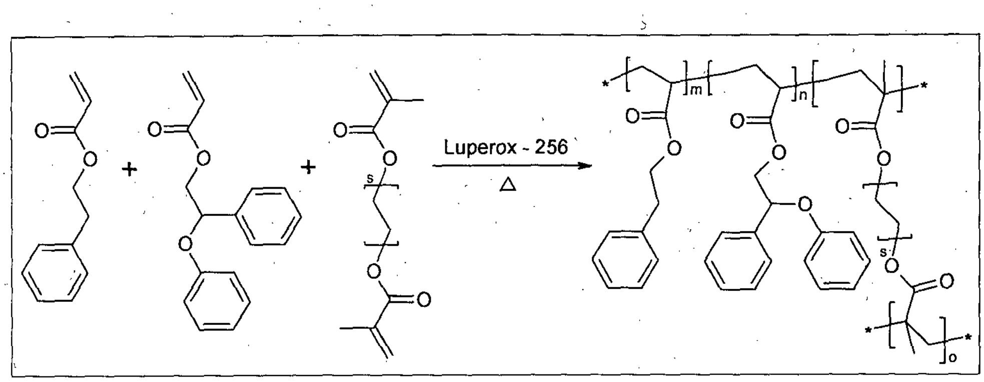


Scheme 1

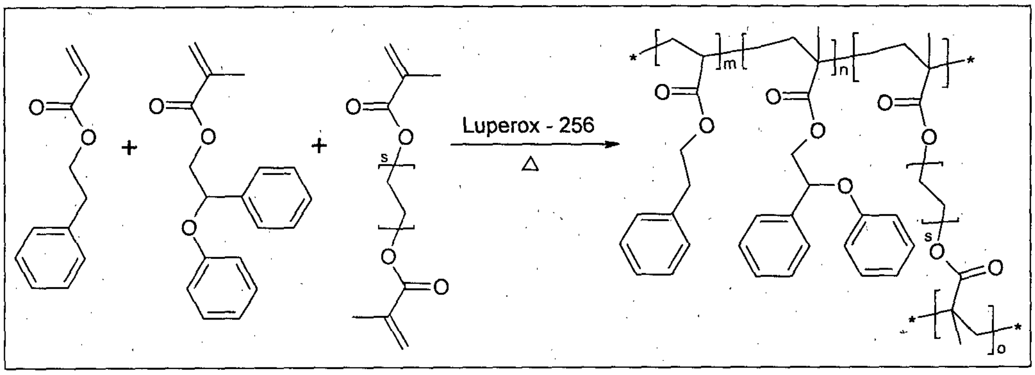


Scheme 2

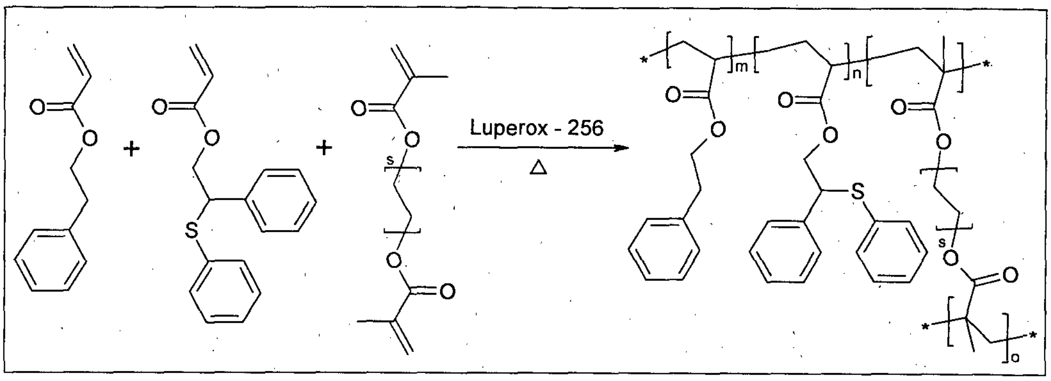


**Scheme 3**

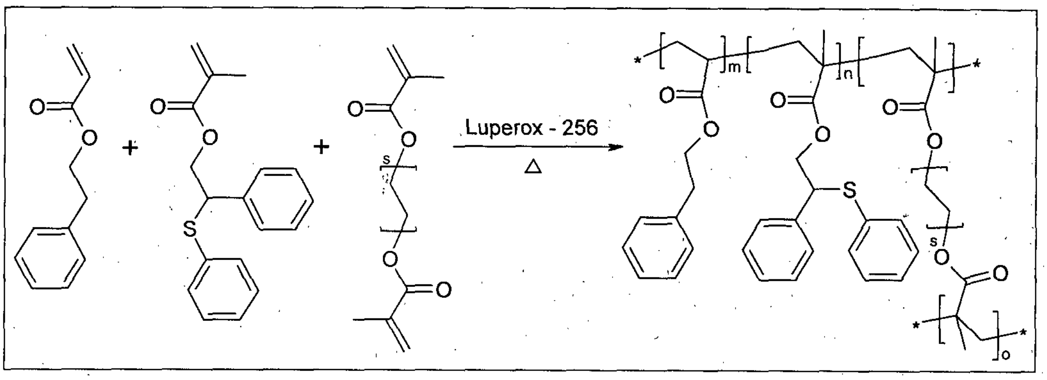


**Scheme 4**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5290892 A
- WO 9724382 A
- WO 9907756 A
- US 2012196951 A
- US 2008139769 A
- WO 1994011764 A
- US 7585900 B
- US 5331073 A
- US 5403901 A
- US 5674960 A
- EP 1030194 A
- US 6653422 B
- US 5693095 A
- US 5433746 A
- US 2008021129 A
- US 2012053313 A
- WO 2004029675 A
- US 2005258096 A
- US 2004031275 A
- US 4304895 A
- US 4528311 A
- US 5814680 A